# EUROPEAN PATENT APPLICATION

(11) **EP 4 249 477 A1**
(43) Date of publication of application: **27.09.2023**
(21) Application number: 21894841.2
(22) Date of filing: 10.09.2021
(51) Int. Cl.: C07D 307/77, C07D 407/12, C07D 409/12, C07D 333/50, C07D 333/76, C07C 211/61, H01L 51/00, H01L 51/50

(54) **COMPOUND AND ORGANIC LIGHT-EMITTING DEVICE COMPRISING SAME**

(30) Priority: 19.11.2020 KR 20200155675
(71) Applicant: LT Materials Co., Ltd., Yongin-si, Gyeonggi-do 17118 (KR)
(72) Inventor: LEE, Gi-Back, Yongin-si, Gyeonggi-do 17118 (KR); HEO, Yu-Jin, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/012353
(87) International publication number: WO 2022/108068

(57) **Abstract**

The present specification relates to a compound of Chemical Formula 1, and an organic light emitting device including the same.

## Description

### [Technical Field]

The present specification relates to a compound, and an organic light emitting device including the same.

The present specification claims priority to and the benefits of Korean Patent Application No. 10-2020-0155675, filed with the Korean Intellectual Property Office on November 19, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### [Disclosure]

### [Technical Problem]

The present specification is directed to providing a compound, and an organic light emitting device including the same.

### [Technical Solution]

One embodiment of the present specification provides a compound of the following Chemical Formula 1. 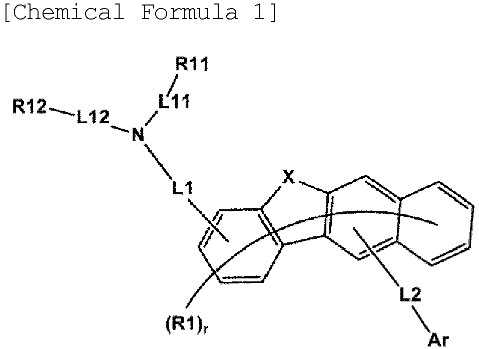 in Chemical Formula 1,
X is O; S; or CR'R",
R' and R" are each independently a substituted or unsubstituted C1 to C60 alkyl group; a substituted or unsubstituted C6 to C60 aryl group; or a substituted or unsubstituted C2 to C60 heteroaryl group,
L1, L2, L11 and L12 are each independently a direct bond; a substituted or unsubstituted C6 to C60 arylene group; or a substituted or unsubstituted C2 to C60 heteroarylene group,
R11, R12 and Ar are each independently a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
R1 is hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r is an integer of 0 to 8, and when 2 or greater, R1s are the same as or different from each other, and
the "substituted or unsubstituted" means being substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; and a C2 to C60 heteroaryl group, or a substituent linking two or more substituents selected from among the substituents illustrated above, or being unsubstituted.

Another embodiment of the present specification provides an organic light emitting device including a first electrode; a second electrode provided opposite to the first electrode; and an organic material layer provided between the first electrode and the second electrode, wherein the organic material layer includes the compound of Chemical Formula 1.

### [Advantageous Effects]

A compound described in the present specification can be used as a material of an organic material layer of an organic light emitting device. The compound is capable of acting as a hole injection material, a hole transfer material, a light emitting material, an electron transfer material, an electron injection material, a charge generation material or the like. Particularly, the compound can be used as a material of a hole transfer layer or an electron blocking layer of an organic light emitting device.

When using the compound of Chemical Formula 1 as a hole transfer layer or electron blocking layer material of an organic light emitting device, an organic light emitting device having superior properties in terms of driving voltage and lifetime can be provided.

Specifically, by bonding a substituent having strengthened hole properties to the amine group-substituted fluorene skeleton in the compound of Chemical Formula 1, the unshared electron pair of the amine improves the flow of holes when used as a hole transfer layer enhancing a hole transfer ability of the hole transfer layer through adjusting band gap and T1 (energy level value in a triplet state) values, and when used as an electron blocking layer, deterioration of a hole transfer material caused by electrons invading the hole transfer layer can be suppressed, and as a result, an effect of providing an organic light emitting device having excellent efficiency can be obtained. In addition, planarity and glass transition temperature of the compound increases improving thermal stability of the compound.

### [Description of Drawings]

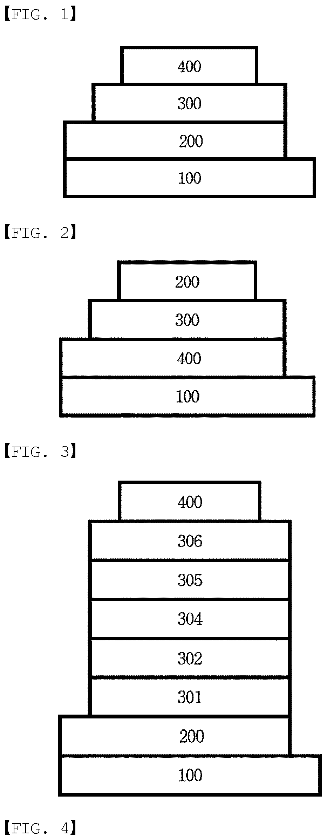

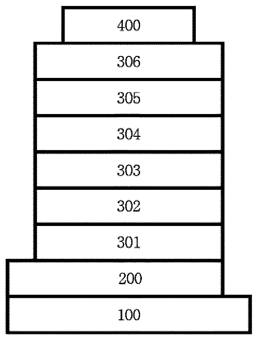

FIG. 1 to FIG. 4 are diagrams each illustrating a lamination structure of an organic light emitting device according to one embodiment of the present specification.

### [Reference Numeral]

- 100:: Substrate
- 200:: Anode
- 300:: Organic Material Layer
- 301:: Hole Injection Layer
- 302:: Hole Transfer Layer
- 303:: Electron Blocking Layer
- 304:: Light Emitting Layer
- 305:: Electron Transfer Layer
- 306:: Electron Injection Layer
- 400:: Cathode

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

A term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; and a C2 to C60 heteroaryl group, or a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 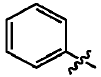 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

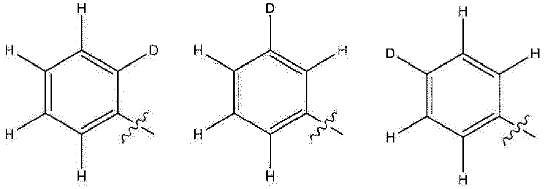

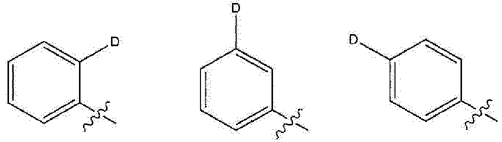

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. When the aryl group is dicyclic or higher, the number of carbon atoms may be from 8 to 60, from 8 to 40 or from 8 to 30. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the terphenyl group may be selected from among the following structures.

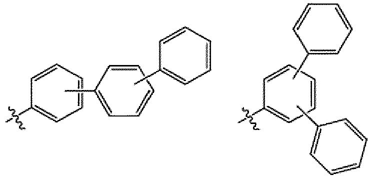

In the present specification, the fluorenyl group may be substituted, and adjacent substituents may bond to each other to form a ring.

When the fluorenyl group is substituted, 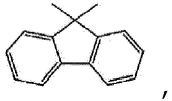 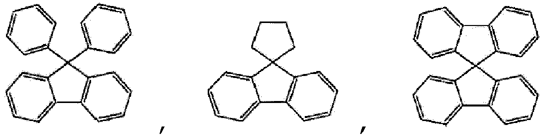 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. When the heteroaryl group is dicyclic or higher, the number of carbon atoms may be from 4 to 60, 4 to 40 or 4 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

In one embodiment of the present specification, X may be O.

In one embodiment of the present specification, X may be S.

In one embodiment of the present specification, X is CR'R", and R' and R" may be each independently a substituted or unsubstituted C1 to C60 alkyl group; or a substituted or unsubstituted C6 to C60 aryl group.

In one embodiment of the present specification, X is CR'R", and R' and R" may be each independently a C1 to C60 alkyl group; or a C6 to C60 aryl group.

In one embodiment of the present specification, X is CR'R", and R' and R" may be each independently a C1 to C30 alkyl group; or a C6 to C30 aryl group.

In one embodiment of the present specification, X is CR'R", and R' and R" may be each independently a C1 to C10 alkyl group; or a C6 to C10 aryl group.

In one embodiment of the present specification, X is CR'R", and R' and R" may be each independently a C1 to C5 alkyl group; or a C6 to C10 aryl group.

In one embodiment of the present specification, X is CR'R", and R' and R" may be each independently a methyl group; or a phenyl group.

In one embodiment of the present specification, Chemical Formula 1 may be represented by any one of the following Chemical Formulae 1-1 to 1-3. 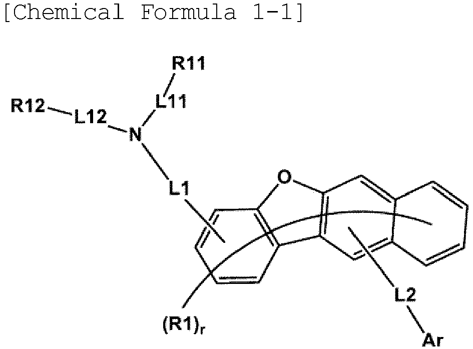 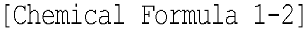 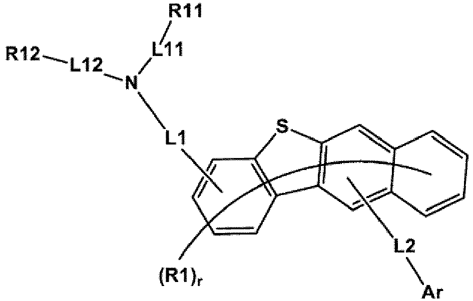 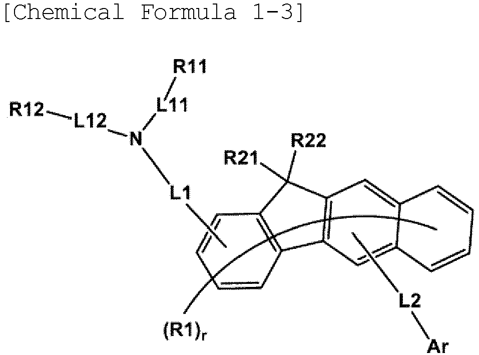 in Chemical Formulae 1-1 to 1-3,
R21 and R22 are each independently a substituted or unsubstituted C1 to C10 alkyl group; or a substituted or unsubstituted C6 to C20 aryl group, and
the rest of the substituents have the same definitions as in Chemical Formula 1.

In one embodiment of the present specification, R21 and R22 of Chemical Formula 1-3 are each independently a substituted or unsubstituted C1 to C5 alkyl group; or a substituted or unsubstituted C6 to C10 aryl group.

In one embodiment of the present specification, R21 and R22 are each independently a C1 to C5 alkyl group; or a C6 to C10 aryl group.

In one embodiment of the present specification, R21 and R22 are each independently a methyl group; or a phenyl group.

In one embodiment of the present specification, L1 and L2 may be each independently a direct bond; or a C6 to C60 arylene group.

In one embodiment of the present specification, L1 and L2 may all be a direct bond.

In an embodiment of the present specification, L1 is a C6 to C60 arylene group, and L2 may be a direct bond.

In an embodiment of the present specification, L1 is a C6 to C30 arylene group, and L2 may be a direct bond.

In an embodiment of the present specification, L1 is a phenylene group, and L2 may be a direct bond.

In an embodiment of the present specification, L1 is a direct bond, and L2 may be a C6 to C30 arylene group.

In an embodiment of the present specification, L1 is a direct bond, and L2 may be a phenylene group.

In one embodiment of the present specification, L11 and L12 may be each independently a direct bond; or a C6 to C60 arylene group.

In one embodiment of the present specification, L11 and L12 may all be a direct bond.

In an embodiment of the present specification, L11 is a C6 to C60 arylene group, and L12 may be a direct bond.

In an embodiment of the present specification, L11 is a C6 to C30 arylene group, and L12 may be a direct bond.

In an embodiment of the present specification, L11 is a phenylene group, and L12 may be a direct bond.

In one embodiment of the present specification, L11 and L12 may be each independently a C6 to C60 arylene group.

In one embodiment of the present specification, L11 and L12 may be each independently a C6 to C30 arylene group.

In one embodiment of the present specification, L11 and L12 may be each independently a C6 to C10 arylene group.

In one embodiment of the present specification, L11 and L12 may all be a phenylene group.

In one embodiment of the present specification, R11 and R12 are each independently a substituted or unsubstituted C6 to C60 aryl group; or a substituted or unsubstituted C2 to C60 heteroaryl group.

In one embodiment of the present specification, R11 and R12 are each independently a substituted or unsubstituted C6 to C40 aryl group; or a substituted or unsubstituted C2 to C40 heteroaryl group.

In one embodiment of the present specification, R11 and R12 are each independently a substituted or unsubstituted C6 to C30 aryl group; or a substituted or unsubstituted C2 to C20 heteroaryl group.

In one embodiment of the present specification, R11 and R12 are each independently a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; a substituted or unsubstituted dibenzofuran group; or a substituted or unsubstituted dibenzothiophene group.

In one embodiment of the present specification, R11 and R12 are each independently a phenyl group; a biphenyl group; a terphenyl group; a naphthyl group; a fluorenyl group unsubstituted or substituted with an alkyl group or an aryl group; 9,9'-spirobi[fluorene]; a dibenzofuran group; or a dibenzothiophene group.

In one embodiment of the present specification, R11 is a substituted or unsubstituted C6 to C60 aryl group, and R12 may be a substituted or unsubstituted C6 to C60 aryl group; or a C2 to C60 heteroaryl group substituted or unsubstituted and including O or S.

In one embodiment of the present specification, R11 is a substituted or unsubstituted C6 to C30 aryl group, and R12 may be a substituted or unsubstituted C6 to C30 aryl group; or a C2 to C20 heteroaryl group substituted or unsubstituted and including O or S.

In one embodiment of the present specification, R11 is a C6 to C30 aryl group unsubstituted or substituted with an alkyl group or an aryl group, and R12 may be a C6 to C30 aryl group unsubstituted or substituted with an alkyl group or an aryl group; or a C2 to C20 heteroaryl group including O or S.

In one embodiment of the present specification, R11 is a substituted or unsubstituted phenyl group; a substituted or unsubstituted biphenyl group; a substituted or unsubstituted terphenyl group; a substituted or unsubstituted naphthyl group; or a substituted or unsubstituted fluorenyl group.

In one embodiment of the present specification, R11 is a phenyl group; a biphenyl group; a terphenyl group; a naphthyl group; a fluorenyl group unsubstituted or substituted with an alkyl group or an aryl group; or 9,9'-spirobi[fluorene].

In one embodiment of the present specification, R12 is a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; a substituted or unsubstituted dibenzofuran group; or a substituted or unsubstituted dibenzothiophene group.

In one embodiment of the present specification, R12 is a phenyl group; a biphenyl group; a terphenyl group; a naphthyl group; a fluorenyl group unsubstituted or substituted with an alkyl group or an aryl group; 9,9'-spirobi[fluorene]; a dibenzofuran group; or a dibenzothiophene group.

In one embodiment of the present specification, Ar is a substituted or unsubstituted C6 to C60 aryl group; or a substituted or unsubstituted C2 to C60 heteroaryl group.

In one embodiment of the present specification, Ar is a substituted or unsubstituted C6 to C40 aryl group; or a substituted or unsubstituted C2 to C40 heteroaryl group.

In one embodiment of the present specification, Ar is a substituted or unsubstituted C6 to C20 aryl group; or a substituted or unsubstituted C2 to C20 heteroaryl group.

In one embodiment of the present specification, Ar is a C6 to C20 aryl group; or a C2 to C20 heteroaryl group.

In one embodiment of the present specification, Ar is a C6 to C20 aryl group; or a C2 to C20 heteroaryl group including O or S.

In one embodiment of the present specification, Ar is a phenyl group; a biphenyl group; a terphenyl group; a naphthyl group; a triphenylenyl group; a dibenzofuran group; or a dibenzothiophene group.

In one embodiment of the present specification, Ar is not substituted with an amine group. Herein, examples of the amine group may include -NH₂; a monoalkylamine group; a monoarylamine group; a monoheteroarylamine group; a dialkylamine group; a diarylamine group; a diheteroarylamine group; an alkylarylamine group; an alkylheteroarylamine group; an arylheteroarylamine group and the like.

In one embodiment of the present specification, R1 is hydrogen; deuterium; a substituted or unsubstituted C1 to C60 alkyl group; a substituted or unsubstituted C6 to C60 aryl group; or a substituted or unsubstituted C2 to C60 heteroaryl group.

In one embodiment of the present specification, R1 is hydrogen; or deuterium.

In one embodiment of the present specification, Chemical Formula 1 may be represented by the following Chemical Formula 2. 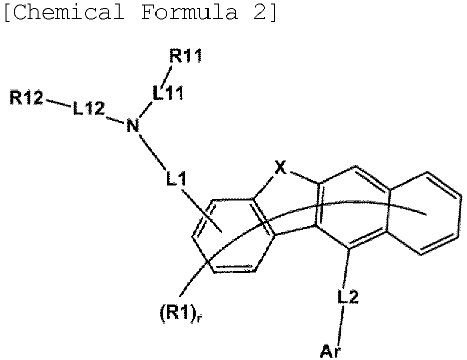 in Chemical Formula 2,
each substituent has the same definition as in Chemical Formula 1.

In one embodiment of the present specification, Chemical Formula 1 may be represented by any one of the following compounds, but is not limited thereto.

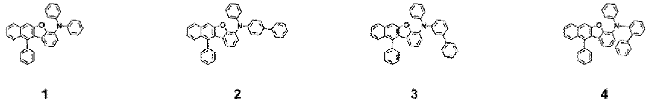

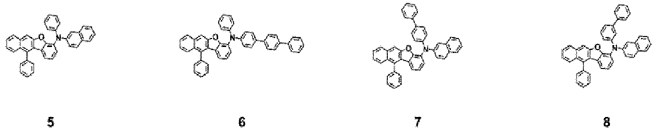

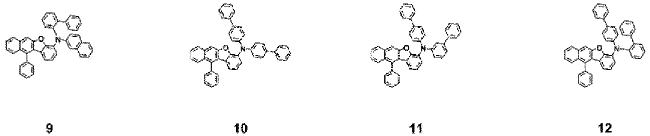

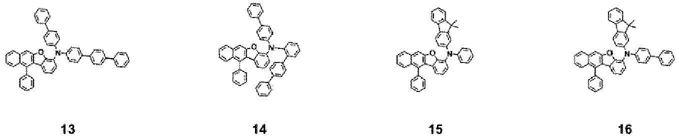

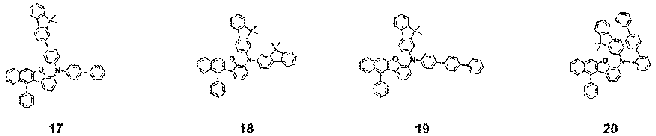

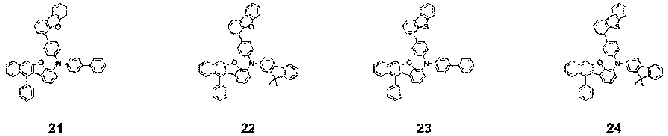

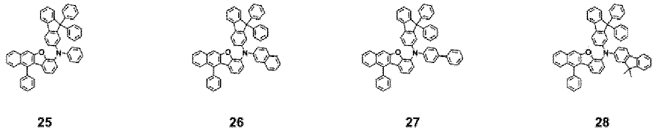

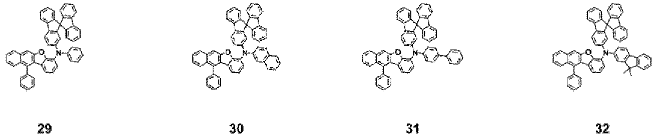

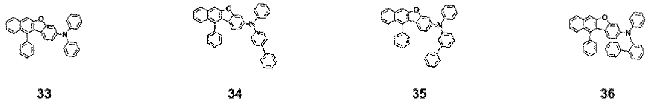

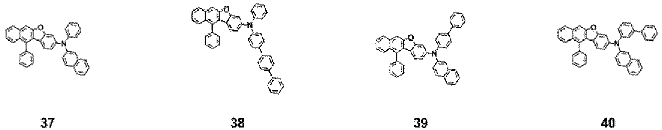

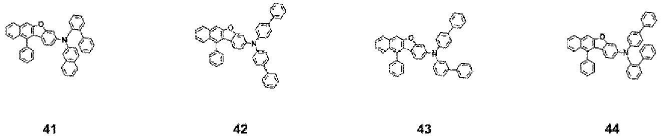

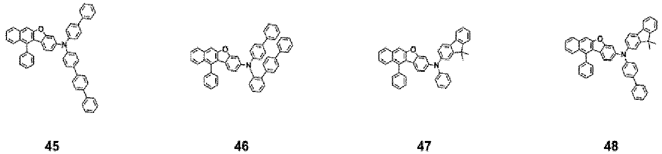

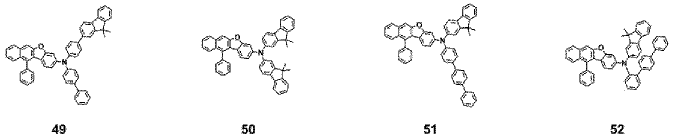

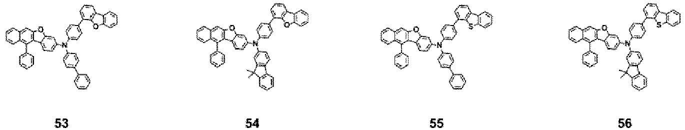

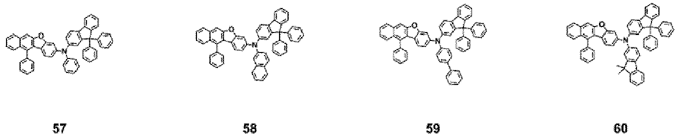

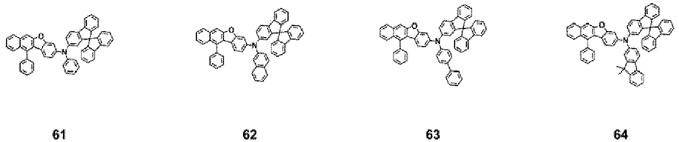

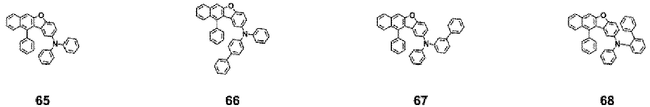

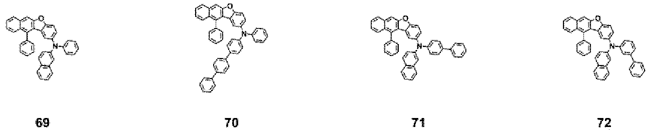

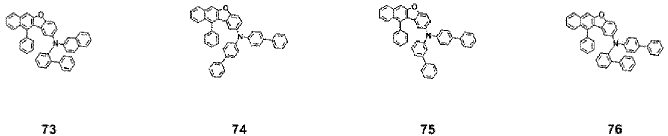

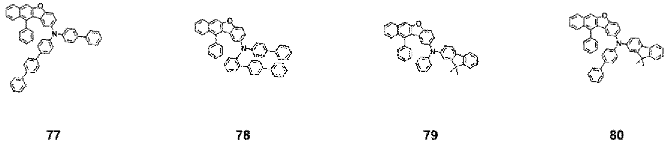

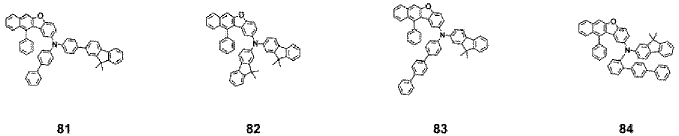

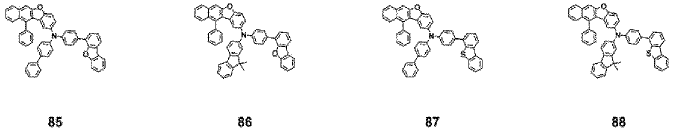

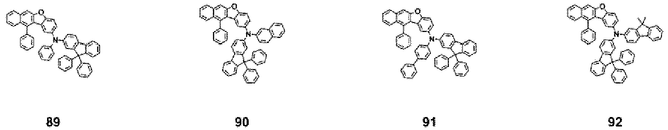

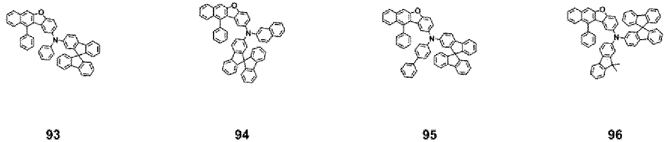

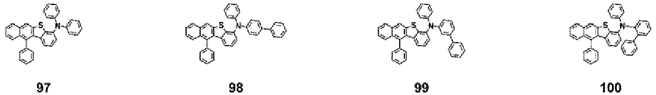

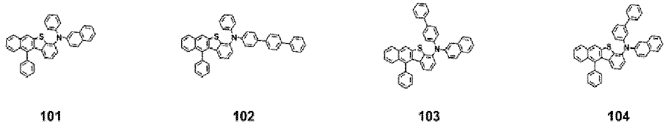

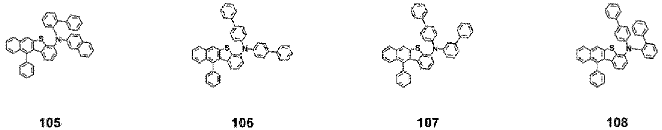

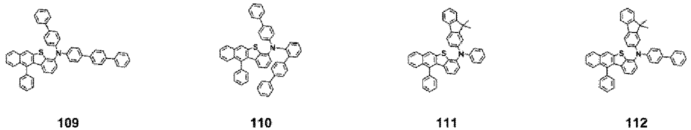

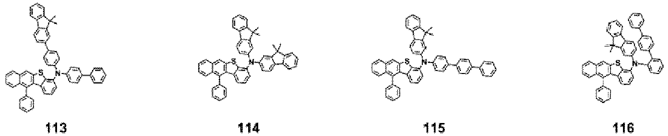

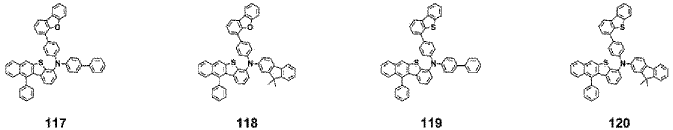

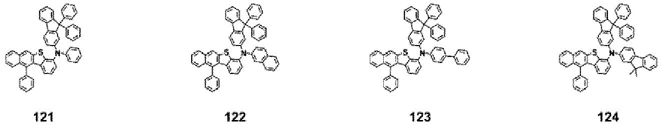

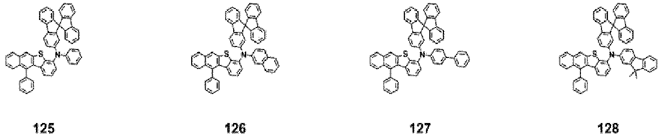

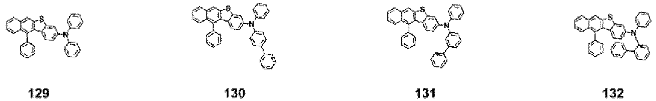

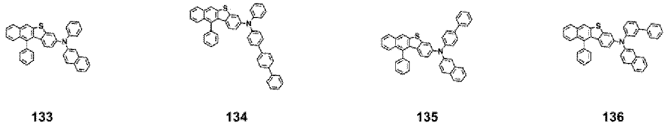

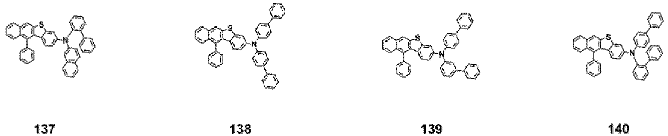

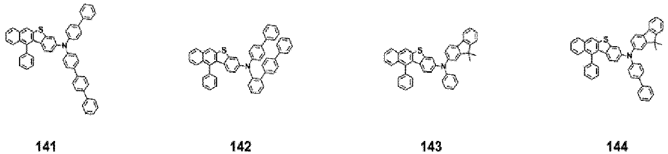

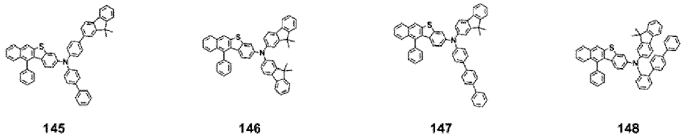

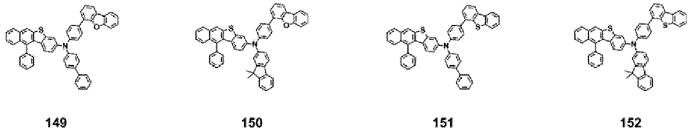

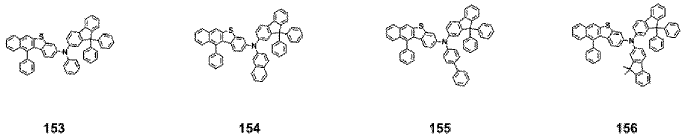

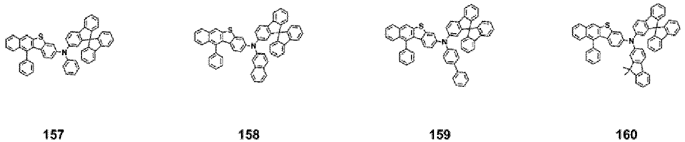

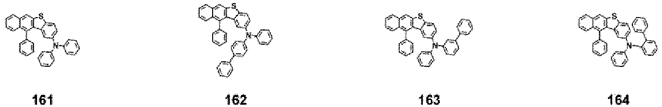

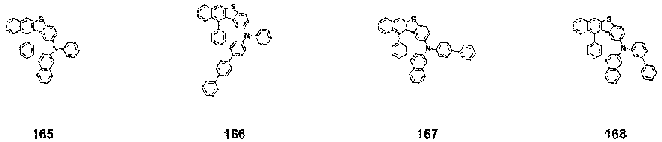

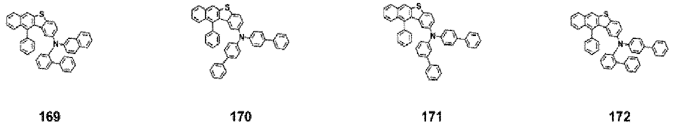

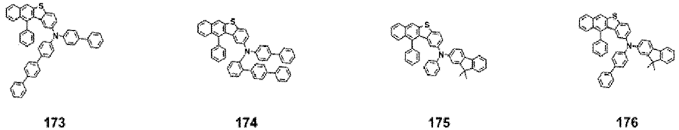

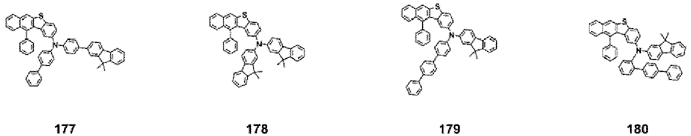

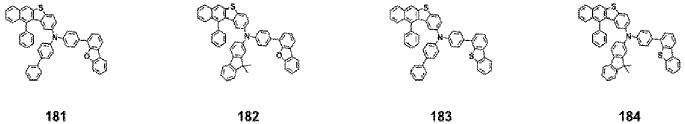

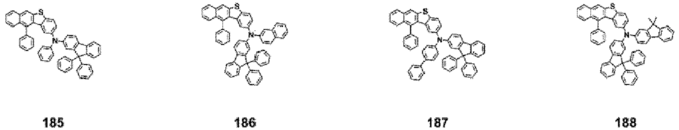

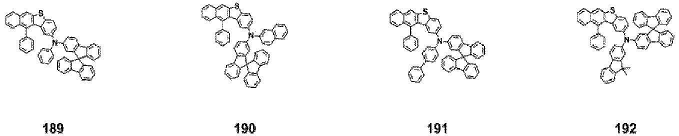

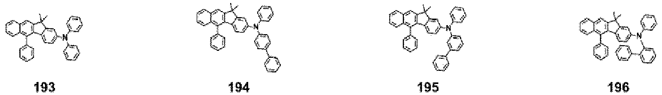

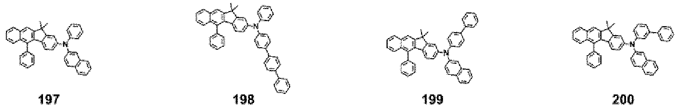

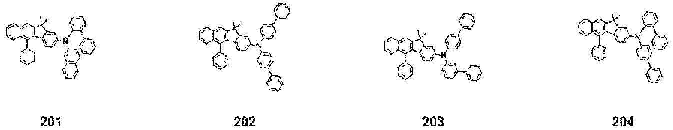

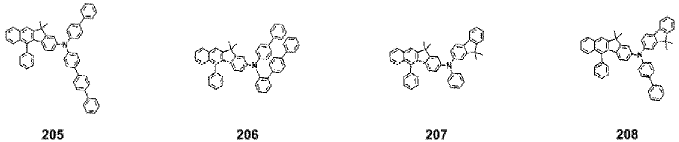

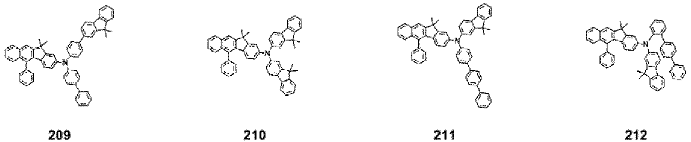

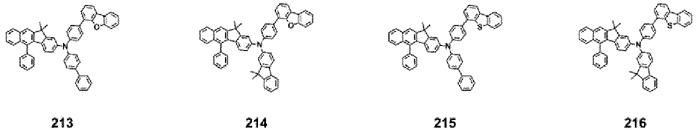

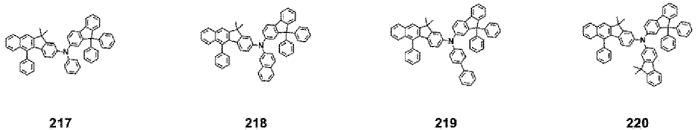

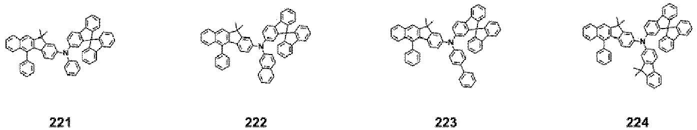

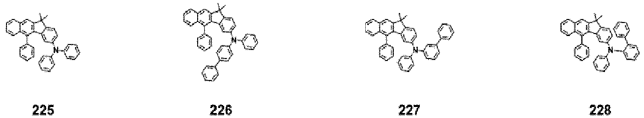

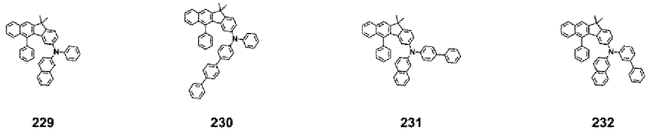

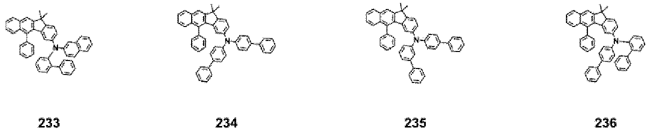

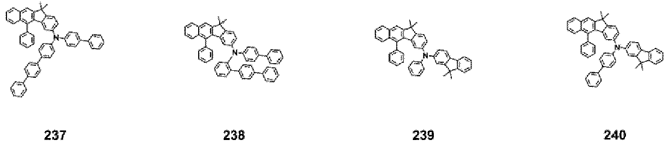

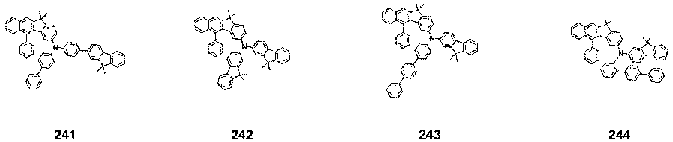

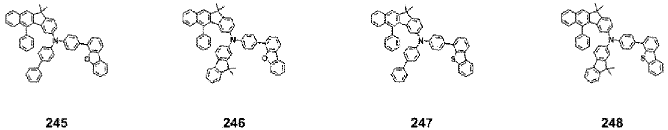

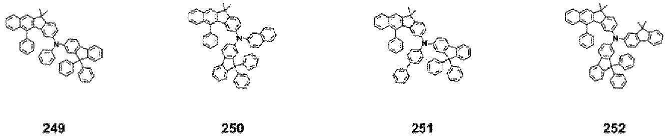

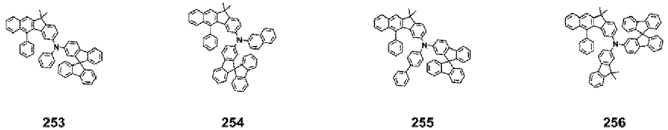

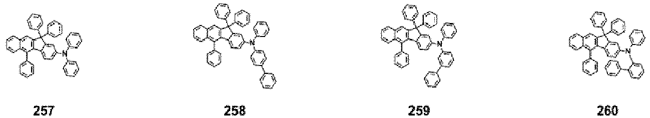

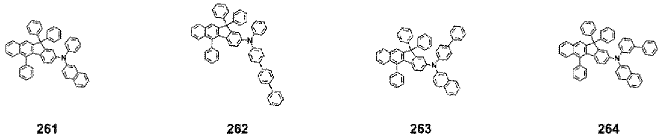

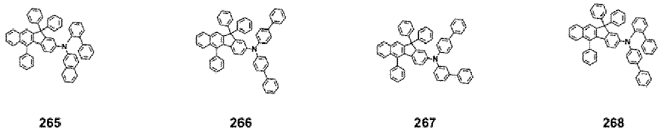

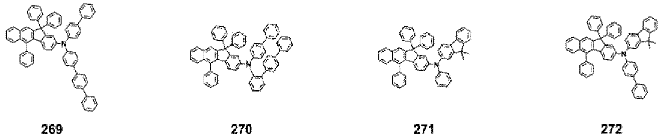

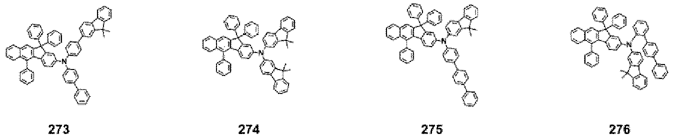

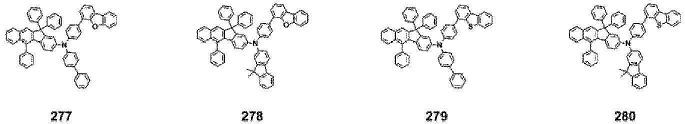

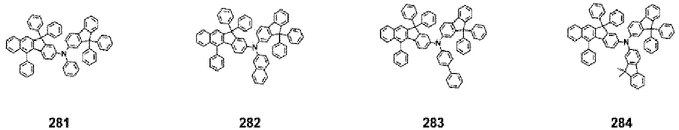

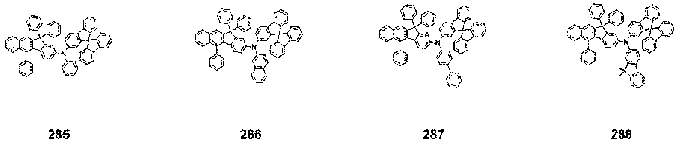

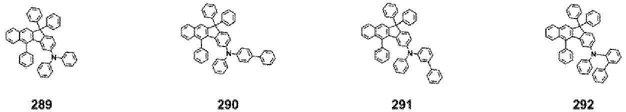

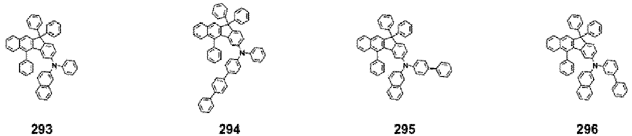

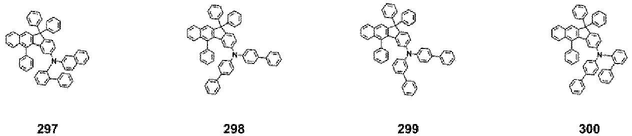

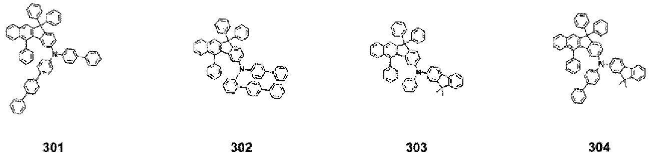

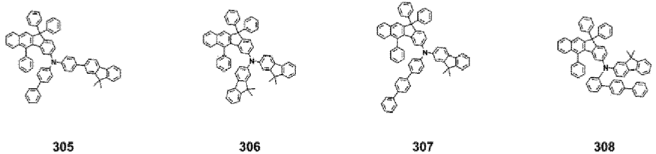

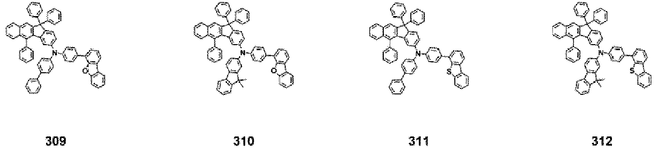

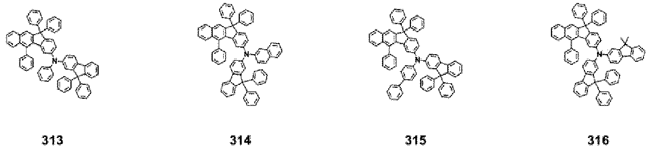

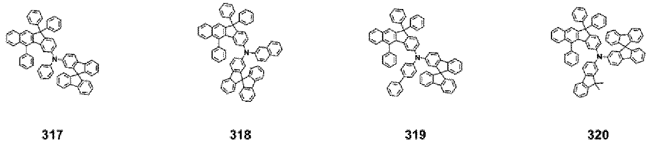

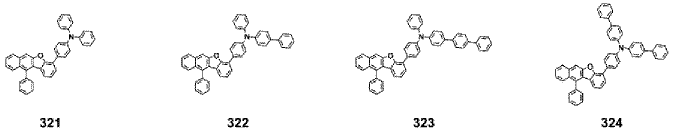

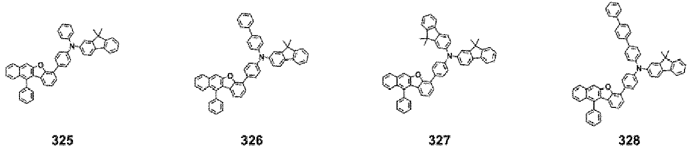

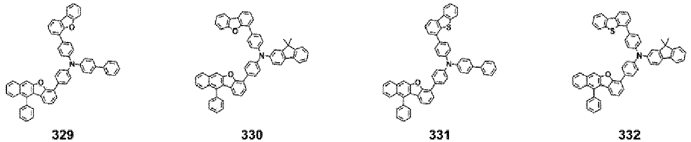

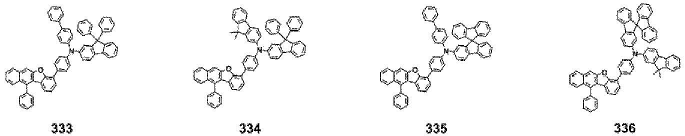

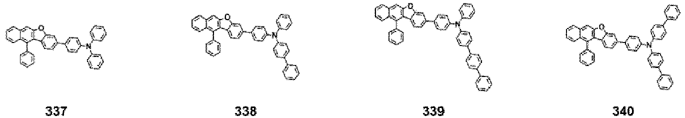

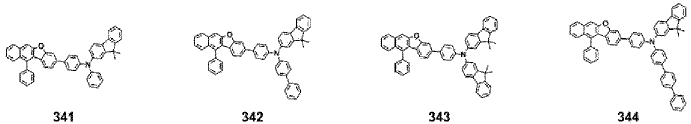

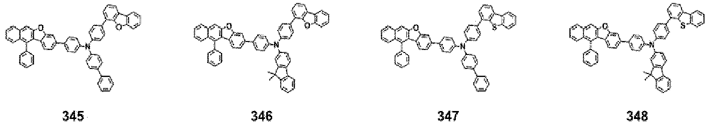

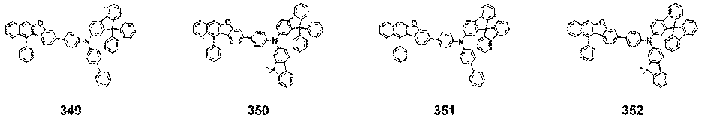

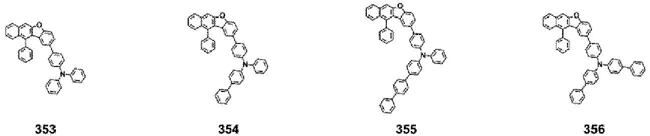

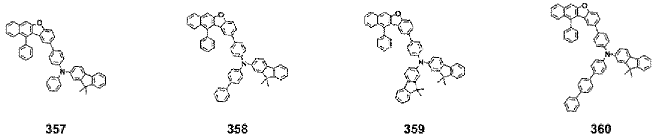

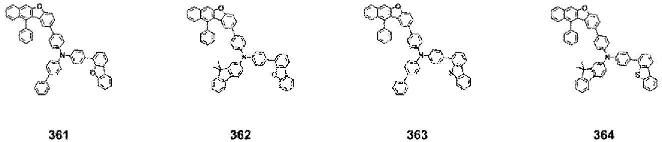

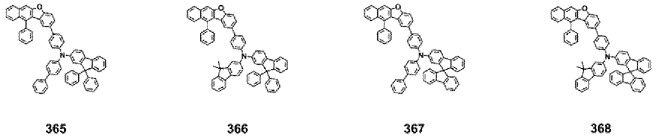

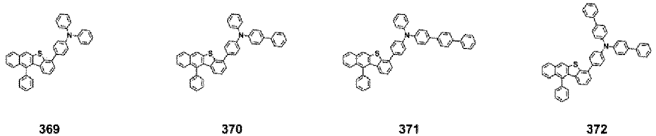

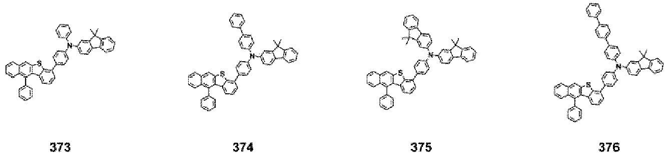

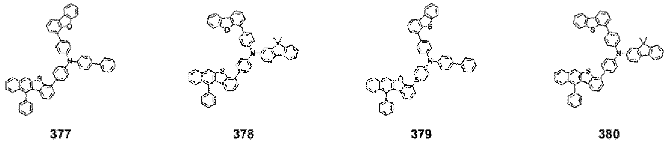

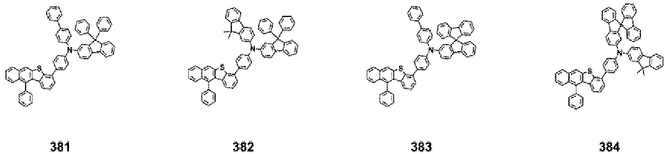

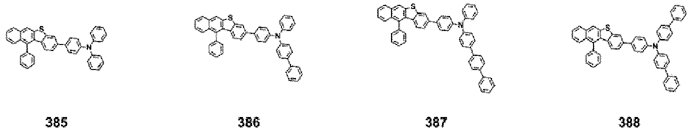

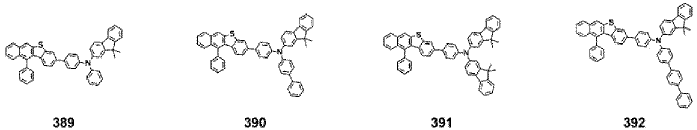

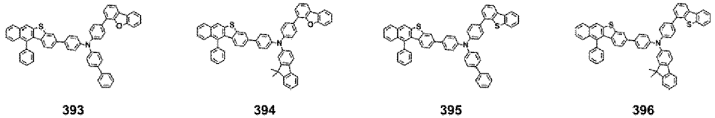

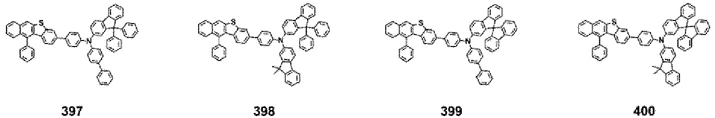

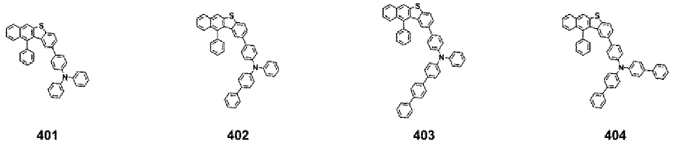

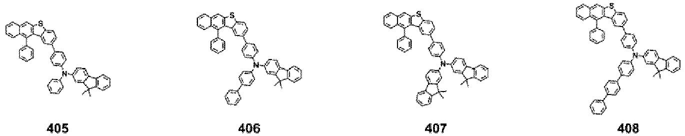

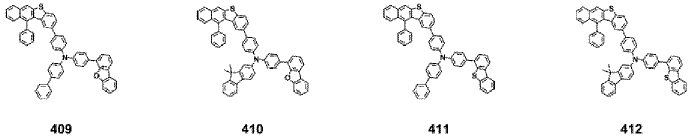

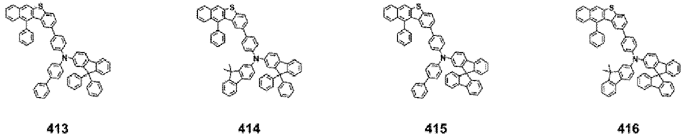

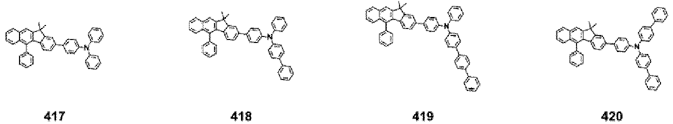

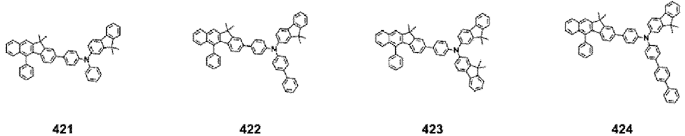

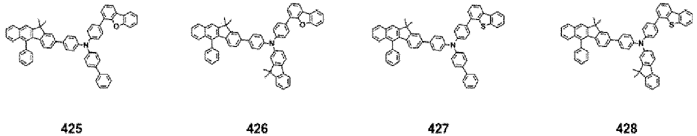

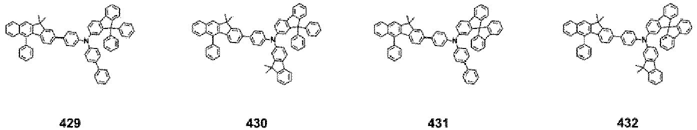

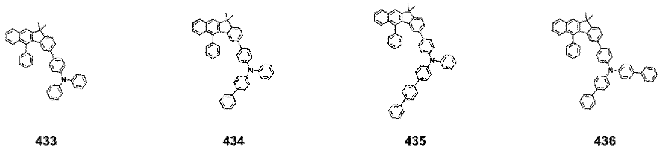

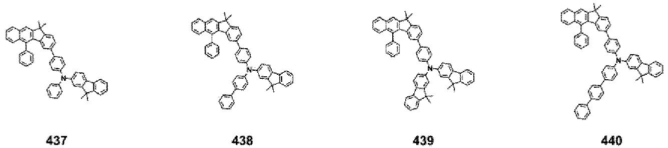

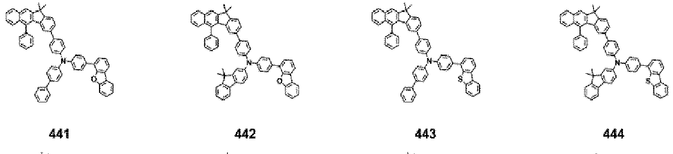

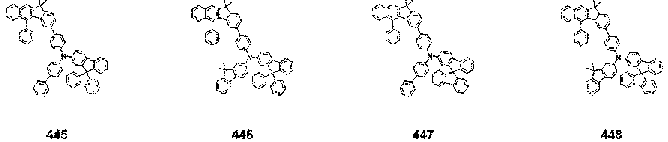

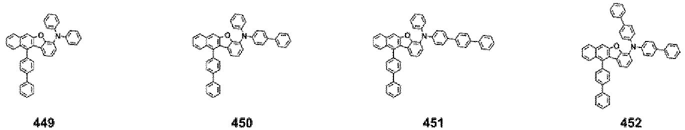

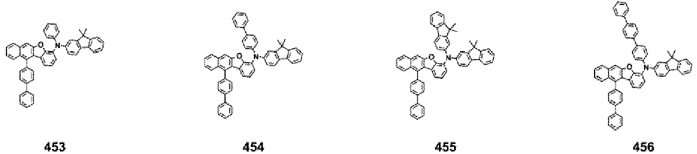

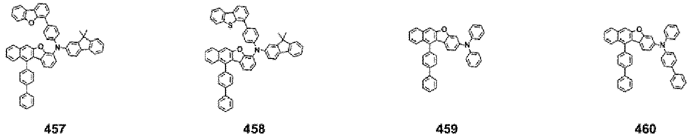

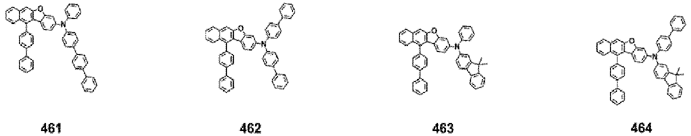

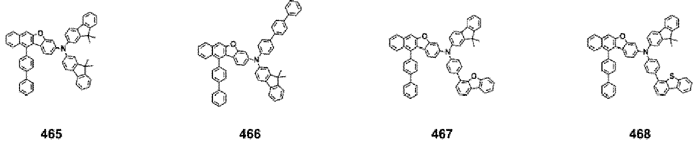

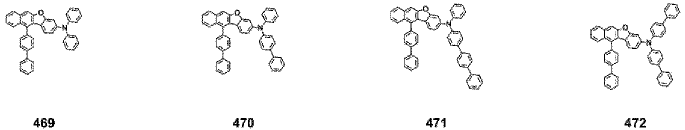

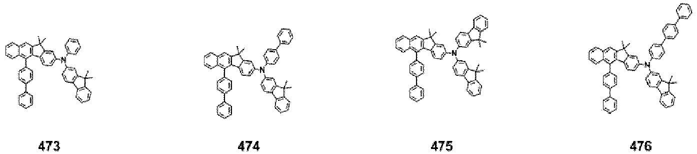

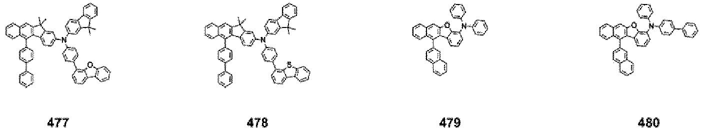

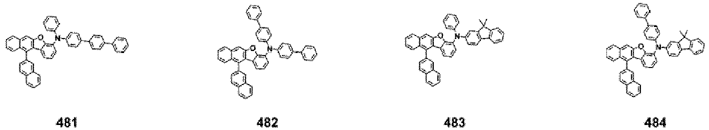

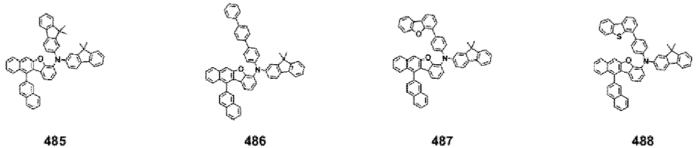

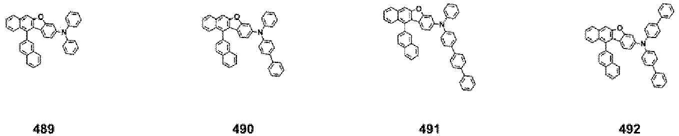

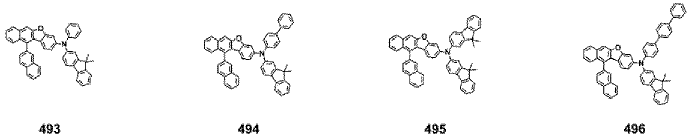

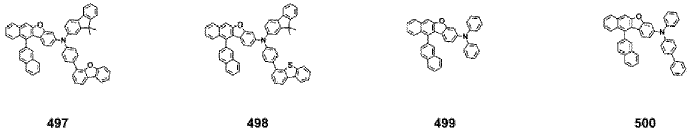

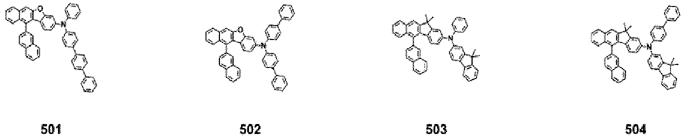

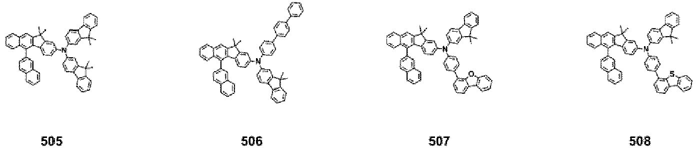

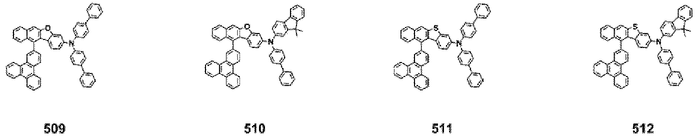

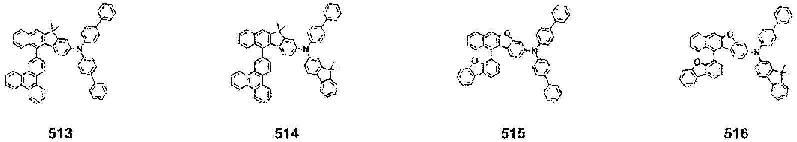

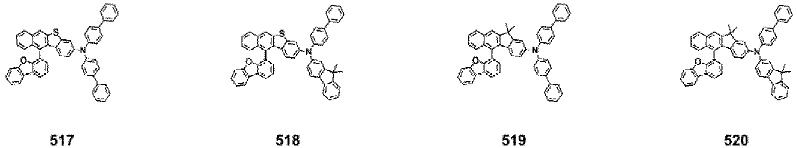

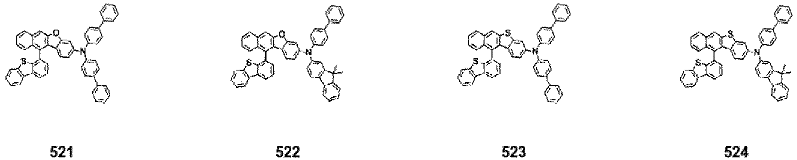

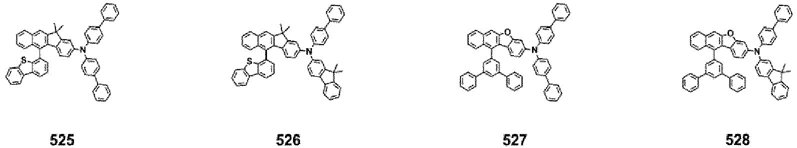

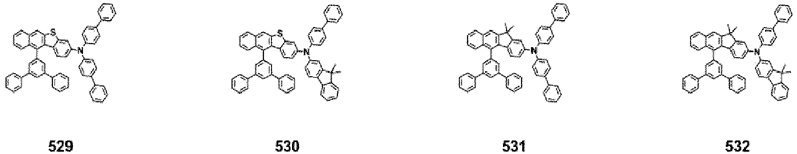

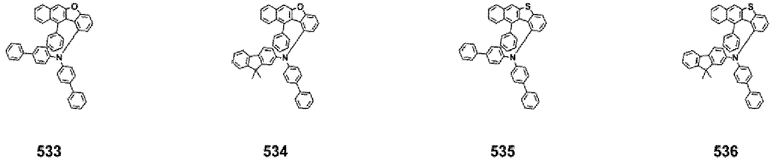

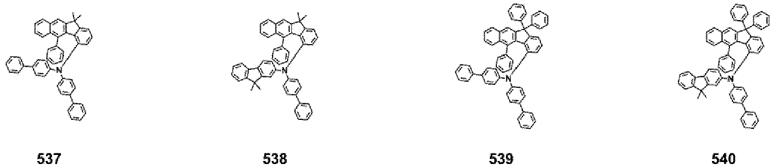

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One embodiment of the present specification provides an organic light emitting device including a first electrode; a second electrode; and one or more organic material layers provided between the first electrode and the second electrode, wherein one or more layers of the organic material layers include the compound of Chemical Formula 1.

In one embodiment of the present specification, the first electrode may be an anode, and the second electrode may be a cathode.

In another embodiment of the present specification, the first electrode may be a cathode, and the second electrode may be an anode.

In one embodiment of the present specification, the organic light emitting device may be a blue organic light emitting device, and the compound of Chemical Formula 1 may be used as a material of the blue organic light emitting device. For example, the compound of Chemical Formula 1 may be included in a hole transfer layer or an electron blocking layer of the blue organic light emitting device.

In one embodiment of the present specification, the organic light emitting device may be a green organic light emitting device, and the compound of Chemical Formula 1 may be used as a material of the green organic light emitting device. For example, the compound of Chemical Formula 1 may be included in a hole transfer layer or an electron blocking layer of the green organic light emitting device.

In one embodiment of the present specification, the organic light emitting device may be a red organic light emitting device, and the compound of Chemical Formula 1 may be used as a material of the red organic light emitting device. For example, the compound of Chemical Formula 1 may be included in a hole transfer layer or an electron blocking layer of the red organic light emitting device.

The organic light emitting device of the present specification may be manufactured using common organic light emitting device manufacturing methods and materials except that one or more organic material layers are formed using the compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present specification, the organic material layer includes a hole transfer layer, and the hole transfer layer may include the compound of Chemical Formula 1.

In the organic light emitting device of the present specification, the organic material layer includes an electron blocking layer, and the electron blocking layer may include the compound of Chemical Formula 1.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 4 illustrate a lamination order of electrodes and organic material layers of the organic light emitting device according to one embodiment of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 and FIG. 4 illustrate cases of the organic material layer being a multilayer. The organic light emitting device according to FIG. 3 includes a hole injection layer (301), a hole transfer layer (302), a light emitting layer (304), an electron transfer layer (305) and an electron injection layer (306), and the organic light emitting device according to FIG. 4 includes a hole injection layer (301), a hole transfer layer (302), an electron blocking layer (303), a light emitting layer (304), an electron transfer layer (305) and an electron injection layer (306). However, the scope of the present application is not limited to such a lamination structure, and as necessary, the layers other than the light emitting layer may not be included, and other necessary functional layers may be further added.

The organic material layer including the compound of Chemical Formula 1 may further include other materials as necessary.

In the organic light emitting device according to one embodiment of the present specification, materials other than the compound of Chemical Formula 1 are illustrated below, however, these are for illustrative purposes only and not for limiting the scope of the present application, and these materials may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, the two or more light emitting materials may be deposited with individual sources of supply or premixed and deposited with one source of supply when used. In addition, fluorescent materials may also be used as the light emitting material, however, phosphorescent materials may also be used. As the light emitting material, materials emitting light by bonding holes and electrons injected from an anode and a cathode, respectively, may be used alone, however, materials having a host material and a dopant material involving together in light emission may also be used.

In one embodiment of the present specification, a compound including anthracene may be used as the host material, however, the host material is not limited thereto.

In one embodiment of the present specification, a pyrene derivative including diamine may be used as the dopant material, however, the dopant material is not limited thereto.

When mixing light emitting material hosts, same series hosts may be mixed, or different series hosts may be mixed. For example, any two or more types of materials among N-type host materials or P-type host materials may be selected and used as a host material of a light emitting layer.

The organic light emitting device according to one embodiment of the present specification may be a top-emission type, a bottom-emission type or a dual-emission type depending on the materials used.

The compound according to one embodiment of the present specification may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### [Preparation Example 1] Preparation of Compound 2

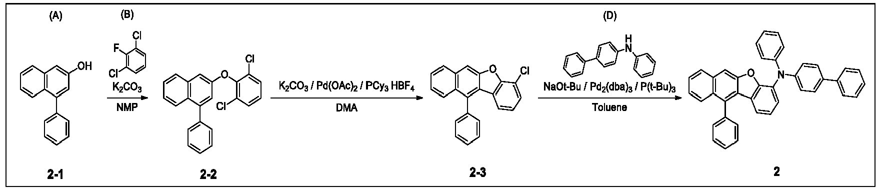

### 1) Preparation of Compound 2-2

NMP (methyl pyrrolidone) (100 ml) was introduced to 4-phenylnaphthalen-2-ol (A) (20 g, 0.090 mol, 1 eq.), 1,3-dichloro-2-fluorobenzene (B) (18 g, 0.108 mol, 1.2 eq.) and K₂CO₃ (25 g, 0.181 mol, 2 eq.), and the mixture was stirred for 12 hours at 160°C. The reaction was terminated by introducing water thereto, and the result was extracted using methylene chloride (MC) and water. After that, moisture was removed with MgSO₄. The result was separated using a silica gel column to obtain Compound 2-2 (14 g) in a 42% yield.

### 2) Preparation of Compound 2-3

Dimethylacetamide (DMA) (140 ml) was introduced to Compound 2-2 (14 g, 0.038 mol, 1 eq.), K₂CO₃ (7.9 g, 0.057 mol, 1.5 eq.), Pd(OAc)₂ (palladium(II) acetate) (0.4 g, 0.0019 mol, 0.05 eq.) and PCy₃HBF₄ (tricyclohexylphosphine-tetrafluoroborate) (1.4 g, 0.0038 mol, 0.1 eq.), and the mixture was stirred for 12 hours at 140°C. The reaction was terminated by introducing water thereto, and the result was extracted using MC and water. After that, moisture was removed with MgSO₄. The result was separated using a silica gel column to obtain Compound 2-3 (8 g) in a 63% yield.

### 3) Preparation of Compound 2

P(t-Bu)₃ (tri-tert-butylphosphine) (0.5 g, 0.0024 mol, 0.1 eq.) and toluene (80 ml) were introduced to Compound 2-3 (8 g, 0.024 mol, 1 eq.), N-phenyl-[1,1'-biphenyl]-4-amine (D) (6.3 g, 0.025 mol, 1.05 eq.), NaOt-Bu (sodium tert-butoxide) (3.4 g, 0.036 mol, 1.5 eq.) and Pd₂(dba)₃ (tris(dibenzylideneacetone)dipalladium(0)) (1.1 g, 0.0012 mol, 0.05 eq.), and the mixture was stirred for 8 hours at 100°C. The reaction was terminated by introducing water thereto, and the result was extracted using MC and water. After that, moisture was removed with MgSO₄. The result was separated using a silica gel column to obtain Compound 2 (8 g) in a 61% yield.

### [Preparation Example 2] Preparation of Compound 194

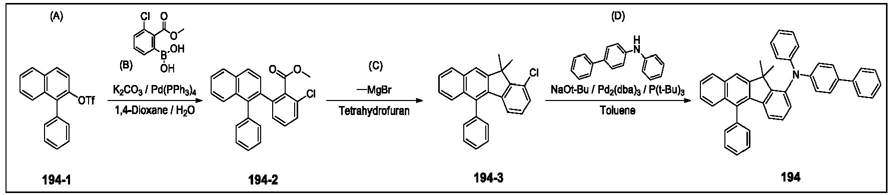

### 1) Preparation of Compound 194-2

1,4-Dioxane (150 ml) and H₂O (45 ml) were introduced to 1-phenylnaphthalen-2-yl trifluoromethanesulfonate (A) (15 g, 0.042 mol, 1 eq.), (3-chloro-2-(methoxycarbonyl)phenyl)boronic acid (B) (10 g, 0.046 mol, 1.1 eq.), K₂CO₃ (12.9 g, 0.094 mol, 2.2 eq.) and Pd(PPh₃)₄ (tetrakis(triphenylphosphine)palladium(0)) (2.4 g, 0.0021 mol, 0.005 eq.), and the mixture was stirred for 6 hours at 100°C. The reaction was terminated by introducing water thereto, and the result was extracted using MC and water. After that, moisture was removed using MgSO₄. The result was separated using a silica gel column to obtain Compound 194-2 (12 g) in a 75% yield.

### 2) Preparation of Compound 194-3

After dissolving Compound 194-2 (12 g, 0.032 mol, 1 eq.) in tetrahydrofuran (THF) (120 ml), methylmagnesium bromide (3 M solution in ether) (D) (32.2 ml, 0.096 mol, 3 eq.) was slowly added thereto at 0°C, and the mixture was stirred for 6 hours at 60°C. The reaction was terminated by introducing water thereto, and the result was extracted using MC and water. After that, moisture was removed using MgSO₄. Boron trifluoride diethyl etherate was added to the reaction material dissolved in MC, and the result was stirred for 4 hours at RT (room temperature). The result was separated using a silica gel column to obtain Compound 194-3 (9 g) in a 79% yield.

### 3) Preparation of Compound 194

Toluene (80 ml) was introduced to Compound 194-3 (9 g, 0.025 mol, 1 eq.), N-phenyl-[1,1'-biphenyl]-4-amine (C) (6.5 g, 0.026 mol, 1.05 eq.), NaOt-Bu (3.7 g, 0.038 mol, 1.5 eq.), Pd₂(dba)₃ (1.1 g, 0.0012 mol, 0.05 eq.) and P(t-Bu)₃ (0.5 g, 0.0024 mol, 0.1 eq.), and the mixture was stirred for 8 hours at 100°C. The reaction was terminated by introducing water thereto, and the result was extracted using MC and water. After that, moisture was removed using MgSO₄. The result was separated using a silica gel column to obtain Compound 194 (10 g) in a 73% yield.

Compounds were synthesized in the same manner as in Preparation Example 1 or 2 except that Intermediates A, B, C and D of the following Table 1 were used instead of (A), (B), (C) and (D).

**[Table 1]**

| Compo und No. | Intermedia te A | Intermedia te B | Intermedia te C | Intermediate D | Yiel d |
|---|---|---|---|---|---|
| 16 | 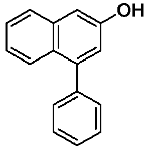 | 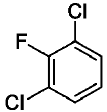 | - | 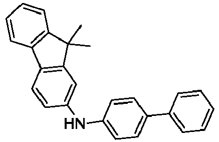 | 52% |
| 42 | 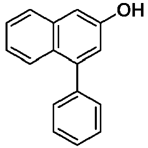 | 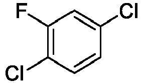 | - | 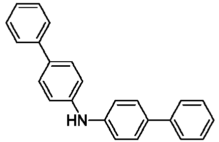 | 50% |
| 50 | 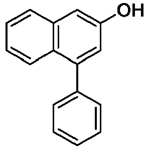 | 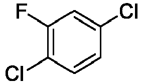 | - | 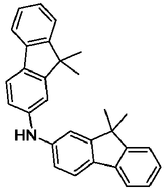 | 56% |
| 60 | 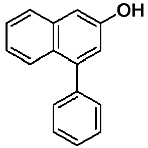 | 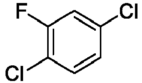 | - | 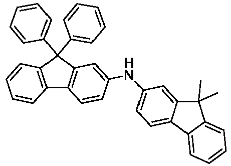 | 61% |
| 138 | 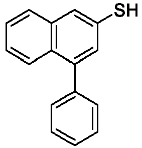 | 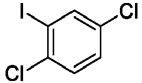 | - | 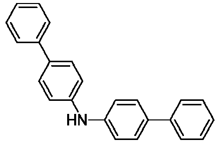 | 42% |
| 144 | 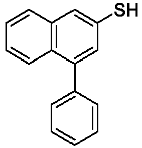 | 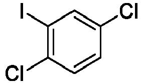 | - | 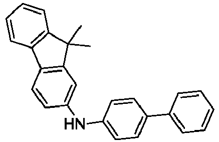 | 44% |
| 202 | 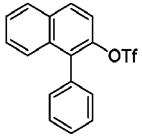 | 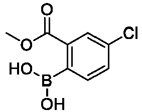 | -MgBr | 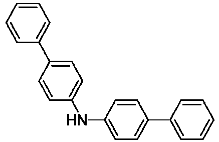 | 53% |
| 212 | 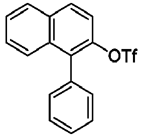 | 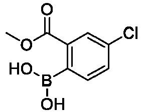 | -MgBr | 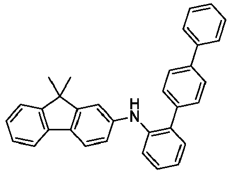 | 48% |
| 240 | 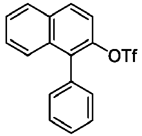 | 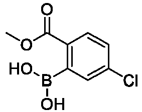 | -MgBr | 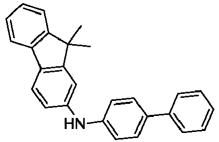 | 55% |
| 256 | 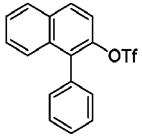 | 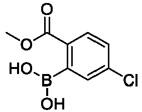 | -MgBr | 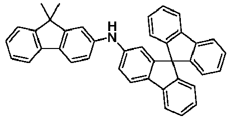 | 52% |
| 268 | 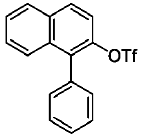 | 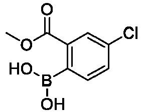 | MgBr | 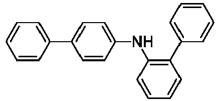 | 47% |
| 272 | 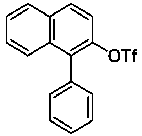 | 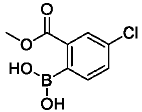 | MgBr | 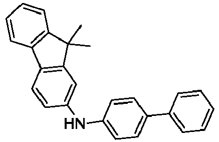 | 60% |
| 277 | 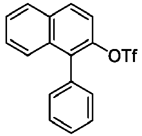 | 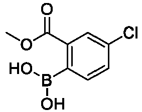 | MgBr | 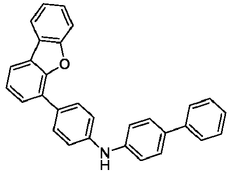 | 63% |
| 289 | 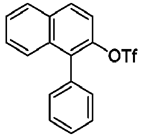 | 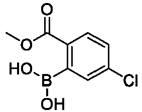 | MgBr | 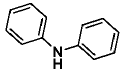 | 67% |
| 294 | 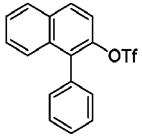 | 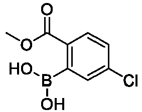 | 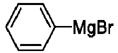 | 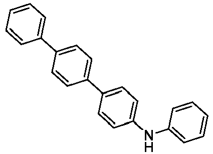 | 59% |
| 340 | 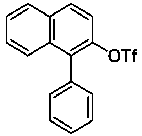 | 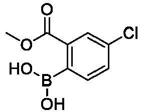 | -MgBr | 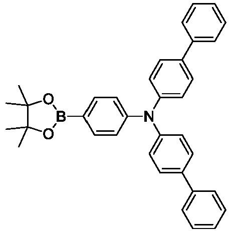 | 60% |
| 420 | 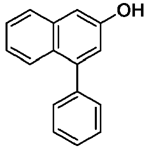 | 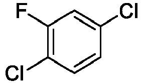 | - | 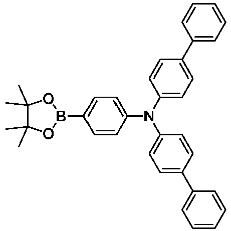 | 55% |
| 454 | 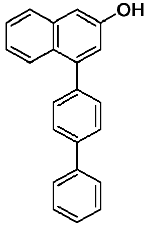 | 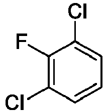 | - | 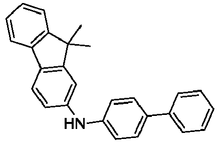 | 53% |
| 464 | 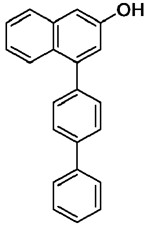 | 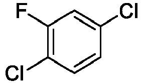 | - | 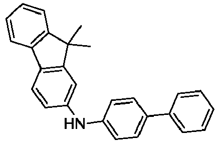 | 57% |
| 473 | 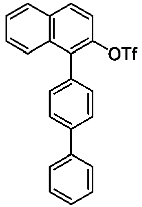 | 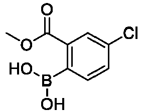 | -MgBr | 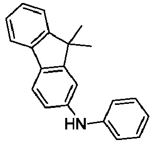 | 59% |
| 483 | 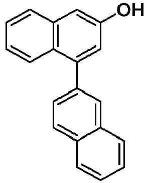 | 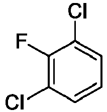 | - | 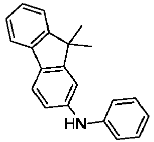 | 51% |
| 495 | 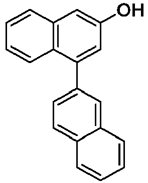 | 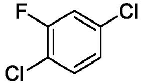 | - | 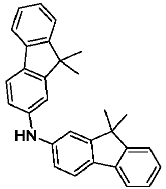 | 48% |

Compounds were prepared in the same manner as in the preparation examples, and the synthesis identification results are shown in Table 2 and Table 3. Table 2 shows measurement values of ¹H NMR (CDCl₃, 200 Mz), and Table 3 shows measurement values of FD-mass spectrometry (FD-Mass: field desorption mass spectrometry).

**[Table 2]**

| Compou nd | ¹H NMR (CDCl₃, 200 Mz) |
|---|---|
| 2 | δ=8.55(1H, d), 8.08(1H, d), 7.51(15H, m), 7.20(3H, m), 7.07 (1H, t), 6.81(1H, t), 6.69(4H, m) 6.39(1H, d) |
| 16 | δ=8.55(1H, d), 8.08(1H, d), 7.87(1H, d), 7.55-7.38(18H, m), 7.28(2H, m), 7.07 (1H, t), 6.75(1H, s), 6.69(2H, m), 6.58(1H, d), 6.39(1H, d), 1.72(6H, s) |
| 42 | δ=8.55(1H, d), 8.08(1H, d), 7.64(1H, d), 7.51(23H, m), 6.69(4H, m) 6.33(1H, d) |
| 50 | δ=8.55(1H, d), 8.08(1H, d), 7.87(2H, d), 7.55(11H, m), 7.38(5H, m), 7.28(2H, m), 6.75(2H, s), 6.58(2H, m) 6.33(1H, d), 1.72 (12H, s) |
| 60 | δ=8.55(1H, d), 8.08(1H, d), 7.87(2H, d), 7.55-7.38(24H, m), 7.11(4H, m), 6.75(2H, s), 6.58(2H, m), 6.33(1H, d), 1.72 (6H, s) |
| 138 | δ=8.55(1H, d), 8.08(1H, d), 7.82(2H, m), 7.55-7.41(21H, m), 7.06 (1H, s), 6.88(1H, d), 6.69(4H, m) |
| 144 | δ=8.55(1H, d), 8.08(1H, d), 7.87(3H, m), 7.62-7.38(17H, m), 7.28(1H, t), 7.06 (1H, s), 6.88(1H, d), 6.69(3H, m), 6.58(1H, d), 1.72(6H, s) |
| 194 | δ=8.56(1H, d), 8.01(2H, m), 7.84(1H, d), 7.54(14H, m), 7.20(2H, m), 6.81(2H, m), 6.63(5H, m), 1.78(6H, s) |
| 202 | δ=8.56(1H, d), 8.01(2H, m), 7.84(1H, d), 7.54(21H, m), 6.81(1H, s), 6.69(5H, m), 1.78(6H, s) |
| 212 | δ=8.56(1H, d), 8.01(2H, m), 7.87(2H, m), 7.51(16H, m), 7.25(5H, m), 7.16(1H, t), 6.87(1H, t), 6.81(1H, s), 6.75(1H, s), 6.64(3H, m), 1.78(6H, s), 1.72(6H, s) |
| 240 | δ=8.56(1H, d), 8.01(2H, m), 7.87(1H, d), 7.62-7.38(18H, m), 7.28(2H, m), 6.75(1H, s), 6.69(2H, m), 6.58(1H, d), 6.44(1H, d), 1.78(6H, s), 1.72(6H, s) |
| 256 | δ=8.56 (1H, d), 8.01(2H, m), 7.87(2H, d), 7.75(2H, d), 7.62-7.16(23H, m), 6.75(2H, s), 6.58(2H, d), 6.44(1H, d), 1.78(6H, s), 1.72 (6H, s) |
| 268 | δ=8.56 (1H, d), 8.01(2H, m), 7.84(1H, d), 7.54-7.26(24H, m), 7.08(7H, m), 6.87(1H, t), 6.81(1H, s), 6.64(4H, m) |
| 272 | δ=8.56 (1H, d), 8.01(2H, m), 7.87(2H, d), 7.55-7.26(24H, m), 7.11(4H, m), 6.81(1H, s), 6.75(1H, s), 6.64(4H, m), 1.72 (6H, s) |
| 277 | δ=8.56 (1H, d), 8.01(2H, m), 7.85(4H, m), 7.66(1H, d), 7.54-7.26(25H, m), 7.11(4H, m), 6.81(1H, s), 6.69(5H, m) |
| 289 | δ=8.56(1H, d), 8.01(2H, m), 7.54-7.26(23H, m), 6.81(2H, m), 6.63(4H, m), 6.44(1H, d) |
| 294 | δ=8.56 (1H, d), 8.01(2H, m), 7.54-7.20(28H, m), 7.11(4H, m), 6.81(1H, t), 6.63(4H, m), 6.44(1H, d) |
| 340 | δ=8.55 (1H, d), 8.08(1H, d), 7.95(1H, d), 7.75(1H, d), 7.64(1H, s), 7.55-7.38(24H, m), 6.69(6H, m) |
| 420 | δ=8.56(1H, d), 8.15(1H, d), 8.01(2H, m), 7.83(1H, s), 7.69(1H, d), 7.55-7.38(23H, m), 6.69(6H, m), 1.78(6H, s) |
| 454 | δ=8.55(1H, d), 8.08(1H, d), 7.87(1H, d), 7.55(18H, m), 7.26(6H, m), 7.07(1H, t), 6.75(1H, t), 6.69(2H, d), 6.58(1H, d), 6.39(1H, d), 1.72(6H, s) |
| 464 | δ=8.55(1H, d), 8.08(1H, d), 7.87(1H, d), 7.55(20H, m), 7.28(5H, m), 6.75(1H, t), 6.69(2H, d), 6.58(1H, d), |
| | 6.33(1H, d), 1.72(6H, s) |
| 473 | δ=8.56(1H, d), 8.01(2H, m), 7.87(2H, d), 7.55(10H, m), 7.28(7H, m), 6.75(3H, m), 6.63(4H, m), 1.78(6H, s), 1.72(6H, s) |
| 483 | δ=8.55(1H, d), 8.00(5H, d), 7.73(1H, d), 7.59(7H, m), 7.38(2H, m), 7.20(4H, m), 7.07 (1H, t), 6.75(2H, m), 6.63(3H, m), 6.39(1H, d), 1.72(6H, s) |
| 495 | δ=8.55(1H, d), 8.08(1H, d), 8.00(2H, m), 7.87(3H, m), 7.73(1H, d), 7.55(10H, m), 7.38(4H, m), 7.28(2H, m), 6.75(2H, s), 6.58(2H, m), 6.33(1H, d), 1.72 (12H, s) |

**[Table 3]**

| Compound | FD-MS | Compound | FD-MS |
|---|---|---|---|
| 2 | m/z=537.21 | 16 | m/z=653.27 |
| 42 | m/z=613.24 | 50 | m/z=693.30 |
| 60 | m/z=817.33 | 138 | m/z=629.22 |
| 144 | m/z=669.25 | 194 | m/z=563.26 |
| 202 | m/z=639.29 | 212 | m/z=755.36 |
| 240 | m/z=679.32 | 256 | m/z=841.37 |
| 268 | m/z=763.32 | 272 | m/z=803.36 |
| 277 | m/z=853.33 | 289 | m/z=611.26 |
| 294 | m/z=763.32 | 340 | m/z=689.27 |
| 420 | m/z=715.32 | 454 | m/z=729.30 |
| 464 | m/z=729.30 | 473 | m/z=679.32 |
| 483 | m/z=627.26 | 495 | m/z=743.32 |

### [Experimental Example]

### <Experimental Example 1>

### 1) Manufacture of Organic Light Emitting Device

### Comparative Example 1

A transparent indium tin oxide (ITO) electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenyl amine (2-TNATA) was introduced to a cell in the vacuum deposition apparatus.

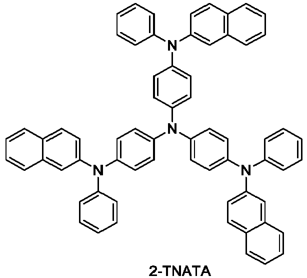

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

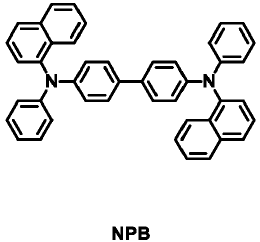

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

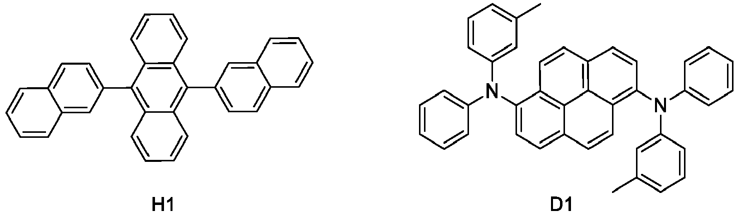

Subsequently, a compound of the following Structural Formula E1 was deposited to a thickness of 300 Å as an electron transfer layer.

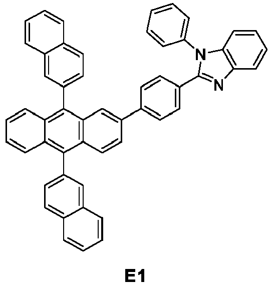

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

### Comparative Examples 2 to 7 and Examples 1 to 24

Organic electroluminescent devices were manufactured in the same manner as in Comparative Example 1 except that compounds shown in the following Table 4 were used instead of NPB used when forming the hole transfer layer in Comparative Example 1.

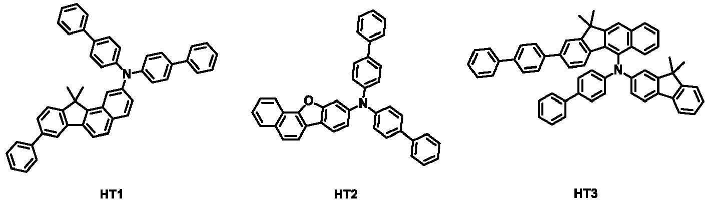

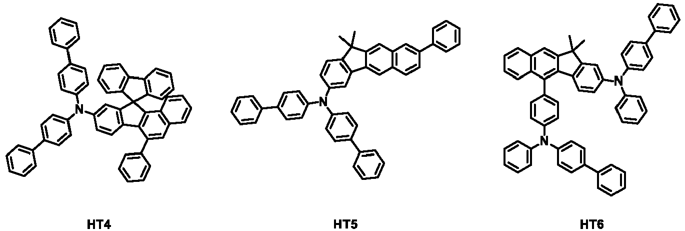

### 2) Evaluation on Organic Light Emitting Device

For each of the organic electroluminescent devices manufactured as above, electroluminescent (EL) properties were measured using M7000 manufactured by McScience Inc., and with the measurement results, T₉₅ was measured when standard luminance was 6000 cd/m² through a lifetime measurement system (M6000) manufactured by McScience Inc..

Results of measuring driving voltage, light emission efficiency, color coordinate (CIE) and lifetime of the blue organic light emitting devices manufactured according to the present disclosure are as shown in Table 4.

**[Table 4]**

| | Compound | Driving Voltage (V) | Light Emission Efficienc y (cd/A) | CIE (x, y) | Lifetim e (T95) |
|---|---|---|---|---|---|
| Example 1 | 2 | 4.80 | 6.60 | (0.134, 0.100) | 45 |
| Example 2 | 16 | 4.78 | 6.64 | (0.134, 0.100) | 49 |
| Example 3 | 42 | 4.75 | 6.69 | (0.134, 0.100) | 47 |
| Example 4 | 50 | 4.73 | 6.70 | (0.134, 0.101) | 48 |
| Example 5 | 60 | 4.79 | 6.68 | (0.134, 0.101) | 50 |
| Example 6 | 138 | 4.81 | 6.61 | (0.134. 0.101) | 45 |
| Example 7 | 144 | 4.83 | 6.63 | (0.134, 0.101) | 44 |
| Example 8 | 194 | 4.78 | 6.73 | (0.134, 0.100) | 49 |
| Example 9 | 202 | 4.81 | 6.70 | (0.134, 0.100) | 49 |
| Example 10 | 212 | 4.84 | 6.68 | (0.134, 0.100) | 46 |
| Example 11 | 240 | 4.79 | 6.60 | (0.134, 0.100) | 47 |
| Example 12 | 256 | 4.76 | 6.81 | (0.134, 0.101) | 49 |
| Example 13 | 268 | 4.88 | 6.74 | (0.134, 0.101) | 50 |
| Example 14 | 272 | 4.83 | 6.70 | (0.134, 0.100) | 46 |
| Example 15 | 277 | 4.75 | 6.69 | (0.134, 0.100) | 44 |
| Example 16 | 289 | 4.69 | 6.68 | (0.134, 0.101) | 46 |
| Example 17 | 294 | 4.82 | 6.70 | (0.134, 0.100) | 47 |
| Example 18 | 340 | 4.76 | 6.71 | (0.134, 0.100) | 45 |
| Example 19 | 420 | 4.77 | 6.68 | (0.134, 0.100) | 45 |
| Example 20 | 454 | 4.80 | 6.73 | (0.134, 0.101) | 48 |
| Example 21 | 464 | 4.81 | 6.66 | (0.134, 0.100) | 49 |
| Example 22 | 473 | 4.79 | 6.68 | (0.134, 0.100) | 47 |
| Example 23 | 483 | 4.78 | 6.51 | (0.134, 0.100) | 45 |
| Example 24 | 495 | 4.77 | 6.69 | (0.134, 0.101) | 48 |
| Comparative Example 1 | NPB | 5.40 | 6.16 | (0.134, 0.101) | 37 |
| Comparative Example 2 | HT1 | 5.22 | 6.24 | (0.134, 0.101) | 36 |
| Comparative Example 3 | HT2 | 5.19 | 6.21 | (0.134, 0.100) | 35 |
| Comparative Example 4 | HT3 | 5.17 | 6.20 | (0.134, 0.100) | 38 |
| Comparative Example 5 | HT4 | 5.19 | 6.19 | (0.134, 0.101) | 35 |
| Comparative Example 6 | HT5 | 5.21 | 6.23 | (0.134, 0.100) | 38 |
| Comparative Example 7 | HT6 | 5.22 | 6.25 | (0.134, 0.100) | 39 |

As seen from the results of Table 4, it was identified that the organic light emitting device using the hole transfer layer material of the blue organic light emitting device of the present disclosure had lower driving voltage, and significantly improved light emission efficiency and lifetime compared to the comparative examples.

When comparing the comparative examples of Table 4 and the compounds of the present disclosure, having an arylamine group is similar, however, there is a difference in that a fluorene group having a substituent is present. A fluorene group having a substituent suppresses pi-pi stacking of the aromatic ring, which prevents degrading device properties caused by increasing a driving voltage of an organic light emitting device. Accordingly, it is considered that the compound of the present disclosure using such a derivative enhances hole transfer properties or stability resulting in superiority in all aspects of driving voltage, efficiency and lifetime.

### <Experimental Example 2>

### 1) Manufacture of Organic Light Emitting Device

### Comparative Example 8

A transparent indium tin oxide (ITO) electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenyl amine (2-TNATA) was introduced to a cell in the vacuum deposition apparatus.

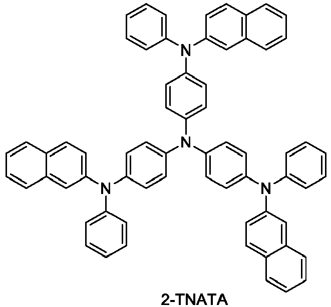

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

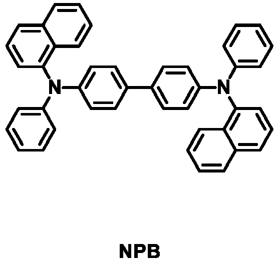

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

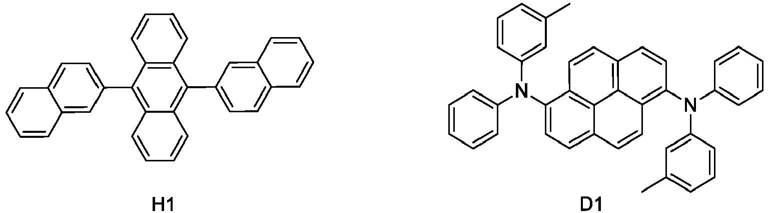

Subsequently, a compound of the following Structural Formula E1 was deposited to a thickness of 300 Å as an electron transfer layer.

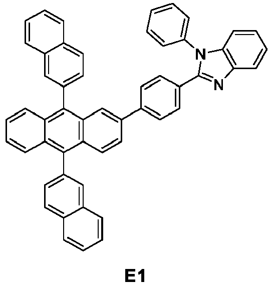

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

### Comparative Examples 9 to 14 and Examples 25 to 48

Organic electroluminescent devices were manufactured in the same manner as in Comparative Example 8 except that an electron blocking layer having a thickness of 50 Å was formed on the hole transfer layer using compounds described in the following Table 5 after forming the hole transfer layer NPB to a thickness of 250 Å in Comparative Example 8.

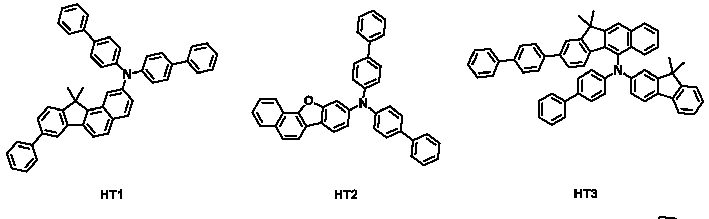

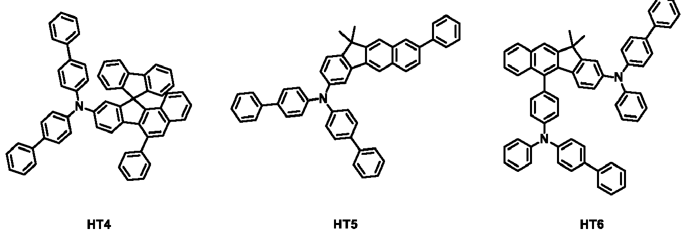

### 2) Evaluation on Organic Light Emitting Device

For each of the organic electroluminescent devices manufactured as above, electroluminescent (EL) properties were measured using M7000 manufactured by McScience Inc., and with the measurement results, T₉₅ was measured when standard luminance was 6000 cd/m² through a lifetime measurement system (M6000) manufactured by McScience Inc..

Results of measuring driving voltage, light emission efficiency, color coordinate (CIE) and lifetime of the blue organic light emitting devices manufactured according to the present disclosure are as shown in the following Table 5.

**[Table 5]**

| | Compo und | Driving Voltage (V) | Light Emission Efficiency (cd/A) | CIE (x, y) | Lifeti me (T₉₅) |
|---|---|---|---|---|---|
| Example 25 | 2 | 4.73 | 6.68 | (0.134, 0.100) | 49 |
| Example 26 | 16 | 4.75 | 6.70 | (0.134, 0.100) | 47 |
| Example 27 | 42 | 4.80 | 6.74 | (0.134, 0.100) | 48 |
| Example 28 | 50 | 4.77 | 6.61 | (0.134, 0.101) | 49 |
| Example 29 | 60 | 4.76 | 6.63 | (0.134, 0.101) | 50 |
| Example 30 | 138 | 4.73 | 6.69 | (0.134, 0.101) | 46 |
| Example 31 | 144 | 4.72 | 6.68 | (0.134, 0.101) | 46 |
| Example 32 | 194 | 4.76 | 6.72 | (0.134, 0.100) | 48 |
| Example 33 | 202 | 4.71 | 6.80 | (0.134, 0.100) | 44 |
| Example 34 | 212 | 4.79 | 6.73 | (0.134, 0.100) | 47 |
| Example 35 | 240 | 4.70 | 6.66 | (0.134, 0.100) | 46 |
| Example 36 | 256 | 4.80 | 6.69 | (0.134, 0.101) | 49 |
| Example 37 | 268 | 4.73 | 6.71 | (0.134, 0.101) | 50 |
| Example 38 | 272 | 4.80 | 6.73 | (0.134, 0.100) | 51 |
| Example 39 | 277 | 4.73 | 6.69 | (0.134, 0.101) | 47 |
| Example 40 | 289 | 4.71 | 6.75 | (0.134, 0.100) | 45 |
| Example 41 | 294 | 4.77 | 6.80 | (0.134, 0.100) | 49 |
| Example 42 | 340 | 4.70 | 6.77 | (0.134, 0.100) | 48 |
| Example 43 | 420 | 4.70 | 6.69 | (0.134, 0.100) | 47 |
| Example 44 | 454 | 4.72 | 6.68 | (0.134, 0.101) | 49 |
| Example 45 | 464 | 4.70 | 6.69 | (0.134, 0.101) | 46 |
| Example 46 | 473 | 4.73 | 6.71 | (0.134, 0.100) | 49 |
| Example 47 | 483 | 4.77 | 6.70 | (0.134, 0.100) | 48 |
| Example 48 | 495 | 4.75 | 6.74 | (0.134, 0.100) | 47 |
| Comparative Example 8 | NPB | 5.31 | 6.11 | (0.134, 0.101) | 36 |
| Comparative Example 9 | HT1 | 5.36 | 6.14 | (0.134, 0.100) | 35 |
| Comparative Example 10 | HT2 | 5.30 | 6.18 | (0.134, 0.100) | 36 |
| Comparative Example 11 | HT3 | 5.33 | 6.16 | (0.134, 0.100) | 37 |
| Comparative Example 12 | HT4 | 5.30 | 6.10 | (0.134, 0.100) | 36 |
| Comparative Example 13 | HT5 | 5.34 | 6.20 | (0.134, 0.101) | 37 |
| Comparative Example 14 | HT6 | 5.29 | 6.22 | (0.134, 0.101) | 38 |

As seen from the results of Table 5, the organic light emitting device using the electron blocking layer material of the blue organic light emitting device of the present disclosure had lower driving voltage, and significantly improved light emission efficiency and lifetime compared to the comparative examples.

When electrons pass through a hole transfer layer and move to an anode without binding in a light emitting layer, efficiency and lifetime of an OLED are reduced. When using a compound having a high LUMO level as an electron blocking layer in order to prevent such a phenomenon, electrons to pass through a light emitting layer and move to an anode are blocked by an energy barrier of the electron blocking layer. Accordingly, holes and electrons are very likely to form excitons increasing possibility of emitting as light in the light emitting layer, and accordingly, the compound of the present disclosure is considered to bring superiority in all aspects of driving voltage, efficiency and lifetime.

Particularly, it was identified that, when using an amine derivative as the hole transfer layer in the compound of Chemical Formula 1 of the present application, the unshared electron pair of the amine improved the flow of holes enhancing a hole transfer ability of the hole transfer layer, and when used as the electron blocking layer, deterioration of the hole transfer material caused by electrons invading the hole transfer layer was suppressed, and in addition, planarity and glass transition temperature of the amine derivative increased by the substituent with strengthened hole properties and the amine site bonding to each other, which increased thermal stability of the compound.

In addition, it was identified that hole transfer ability was enhanced and molecular stability increased as well through adjusting band gap and T1 (energy level value in a triplet state) values, and as a result, driving voltage of the device was lowered, light efficiency was enhanced, and lifetime properties of the device were enhanced by thermal stability of the compound.

## Claims

1. A compound of the following Chemical Formula 1: 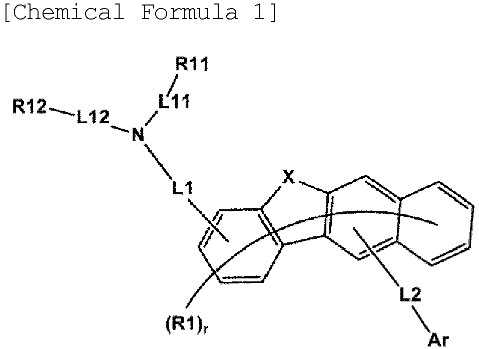 in Chemical Formula 1,
X is O; S; or CR'R",
R' and R" are each independently a substituted or unsubstituted C1 to C60 alkyl group; a substituted or unsubstituted C6 to C60 aryl group; or a substituted or unsubstituted C2 to C60 heteroaryl group,
L1, L2, L11 and L12 are each independently a direct bond; a substituted or unsubstituted C6 to C60 arylene group; or a substituted or unsubstituted C2 to C60 heteroarylene group,
R11, R12 and Ar are each independently a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
R1 is hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r is an integer of 0 to 8, and when 2 or greater, R1s are the same as or different from each other, and
the "substituted or unsubstituted" means being substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; and a C2 to C60 heteroaryl group, or a substituent linking two or more substituents selected from among the substituents illustrated above, or being unsubstituted.

2. The compound of Claim 1, wherein Chemical Formula 1 is represented by any one of the following Chemical Formulae 1-1 to 1-3: 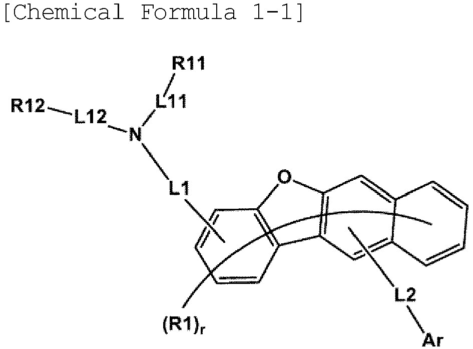 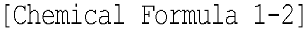 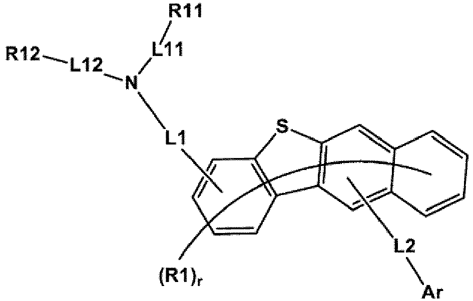 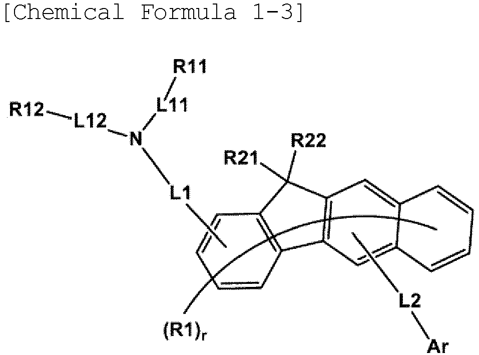 in Chemical Formulae 1-1 to 1-3,
R21 and R22 are each independently a substituted or unsubstituted C1 to C10 alkyl group; or a substituted or unsubstituted C6 to C20 aryl group, and
the rest of the substituents have the same definitions as in Chemical Formula 1.

3. The compound of Claim 1, wherein Chemical Formula 1 is represented by the following Chemical Formula 2: 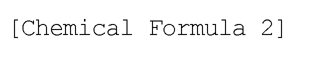 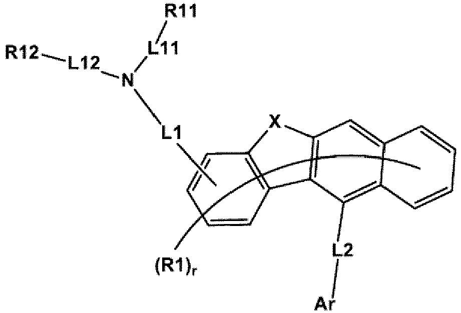 in Chemical Formula 2,
each substituent has the same definition as in Chemical Formula 1.

4. The compound of Claim 1, wherein Ar is a C6 to C20 aryl group; or a C2 to C20 heteroaryl group.

5. The compound of Claim 1, wherein R11 is a substituted or unsubstituted C6 to C60 aryl group, and R12 is a substituted or unsubstituted C6 to C60 aryl group; or a C2 to C60 heteroaryl group substituted or unsubstituted and including O or S.

6. The compound of Claim 1, wherein Chemical Formula 1 is represented by any one of the following compounds:

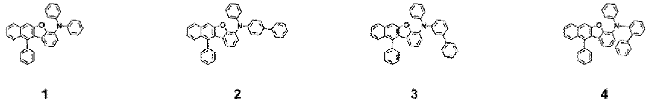

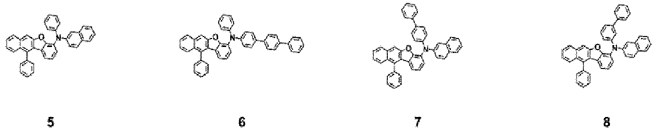

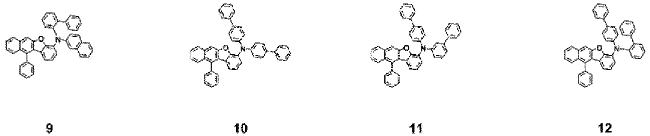

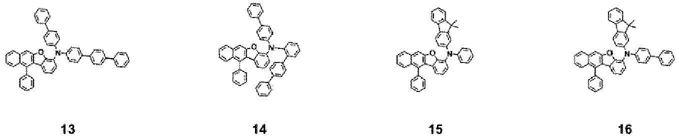

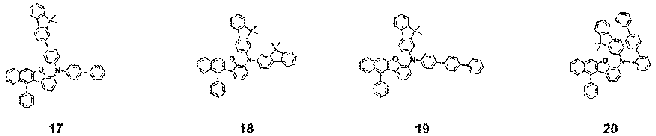

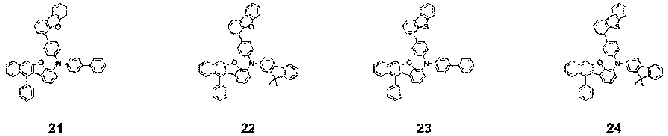

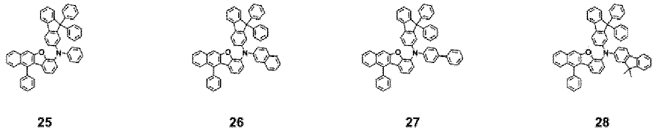

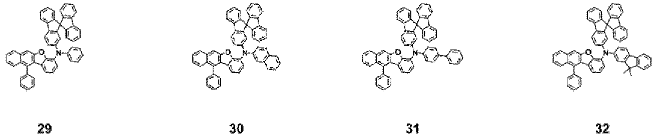

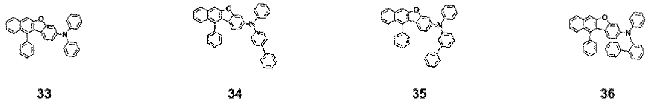

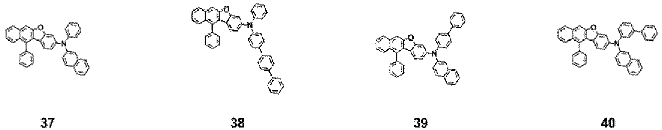

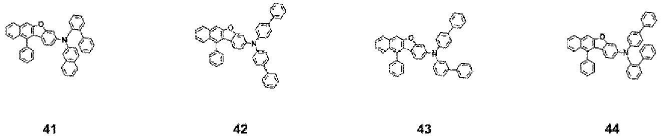

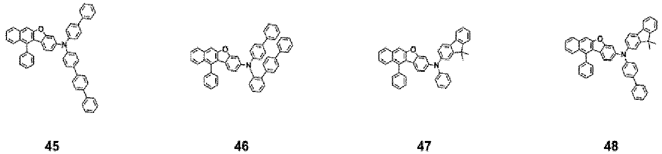

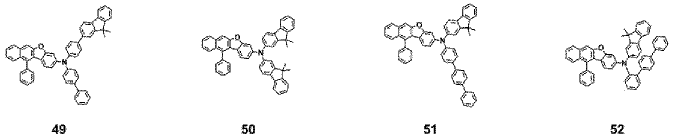

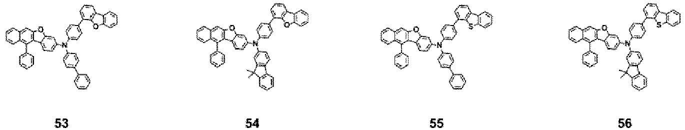

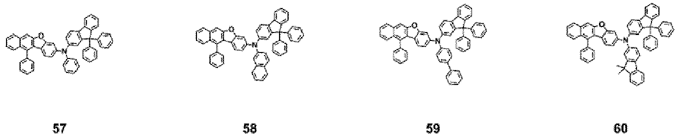

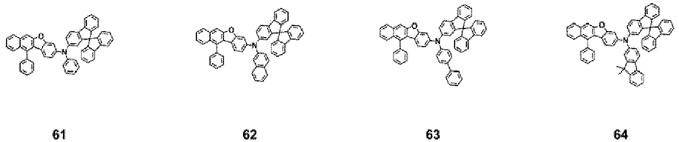

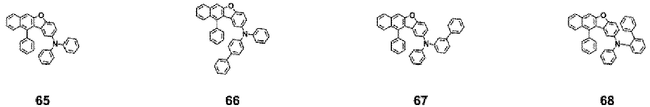

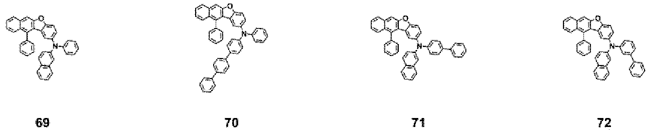

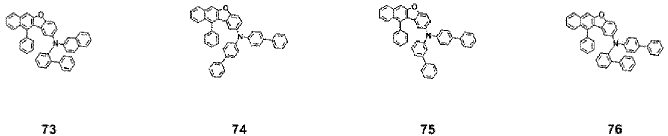

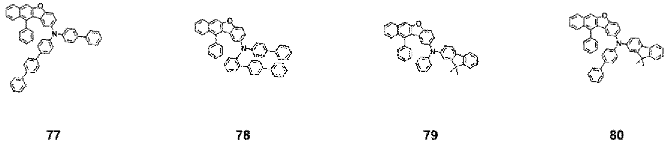

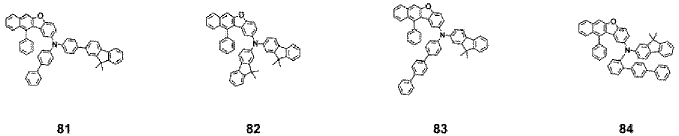

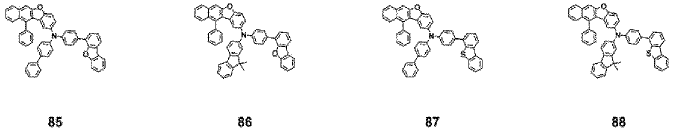

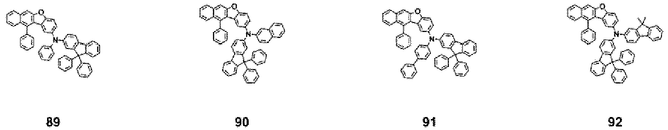

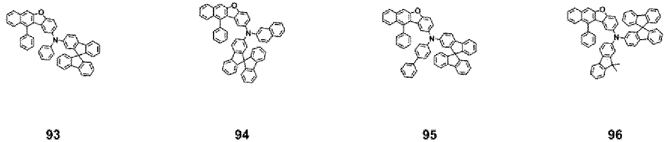

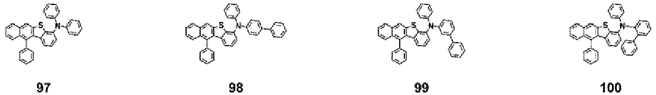

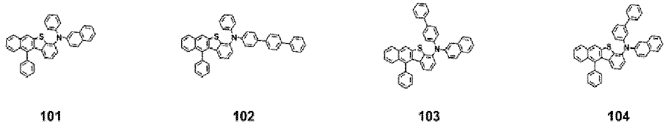

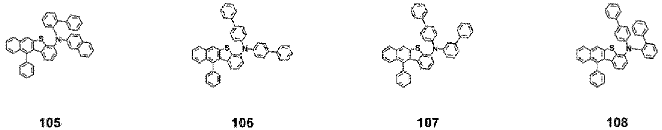

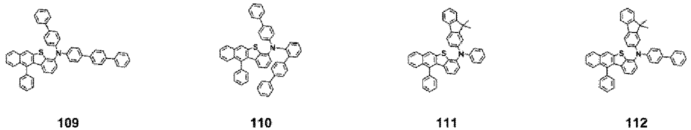

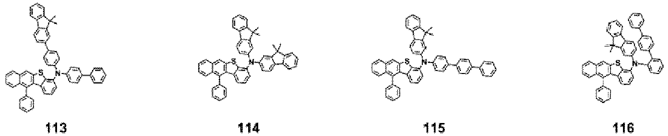

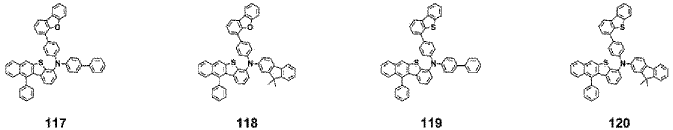

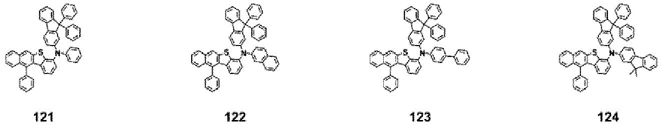

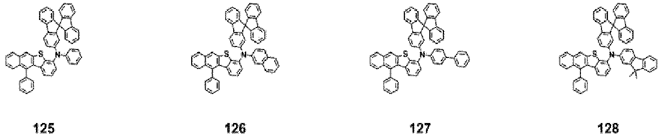

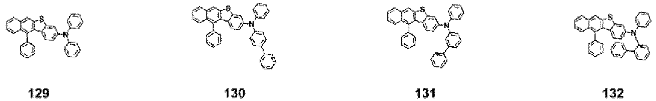

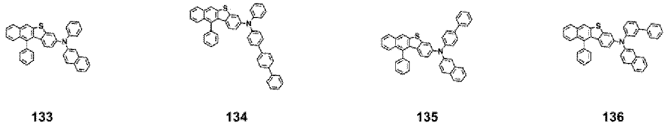

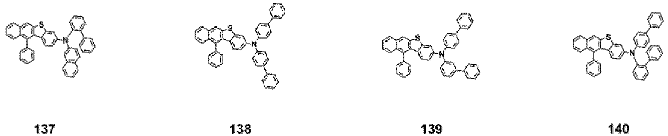

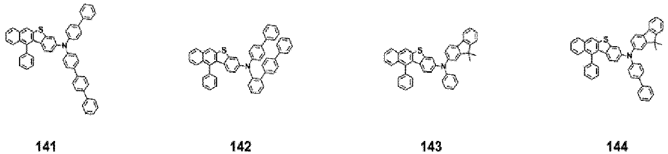

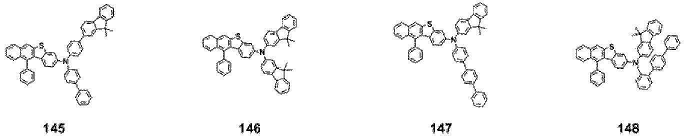

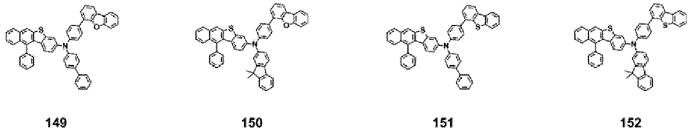

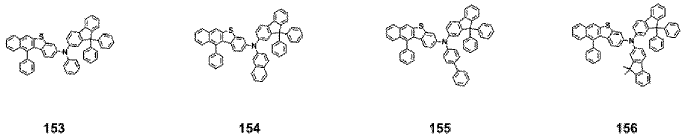

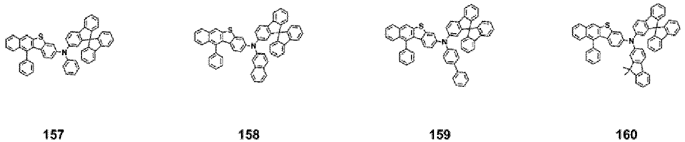

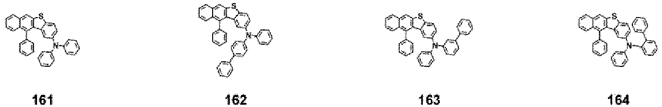

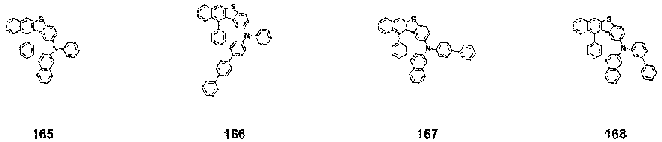

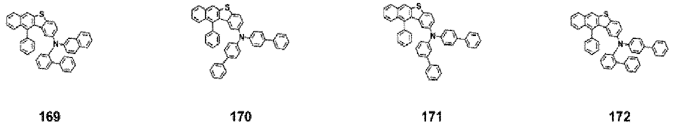

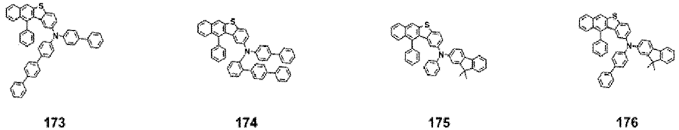

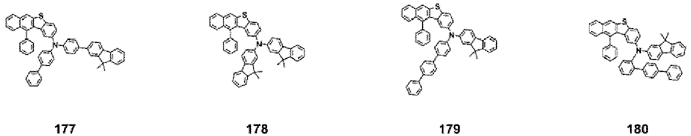

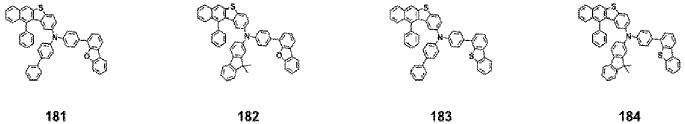

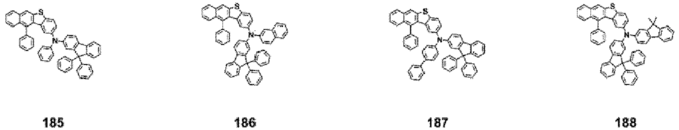

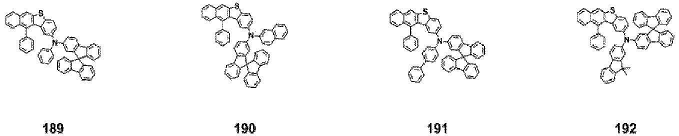

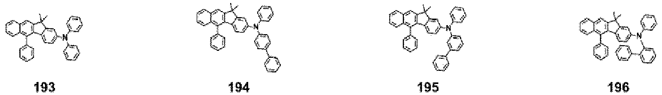

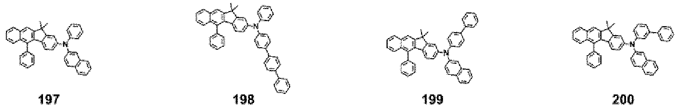

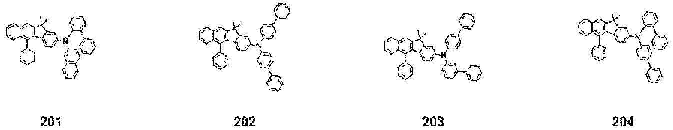

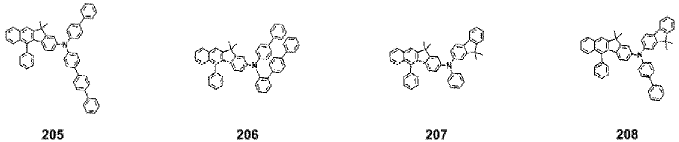

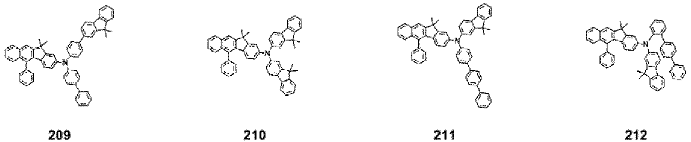

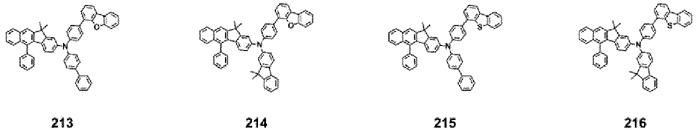

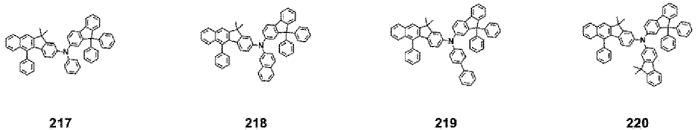

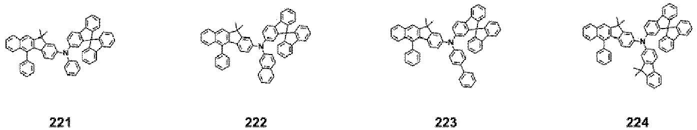

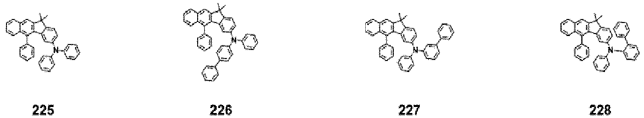

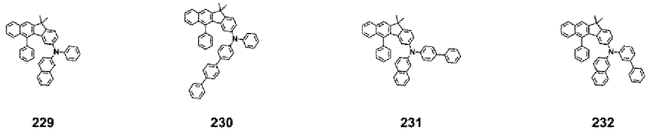

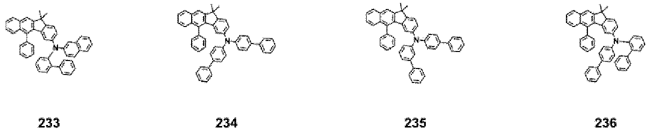

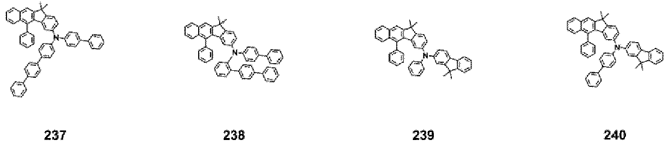

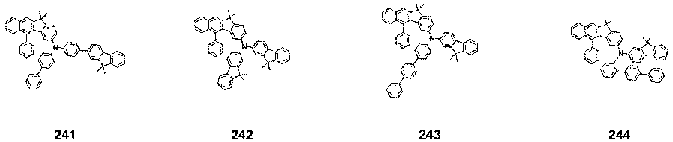

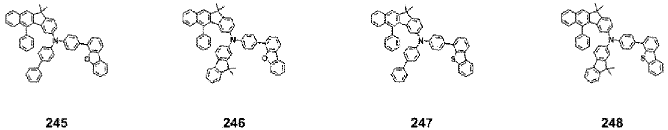

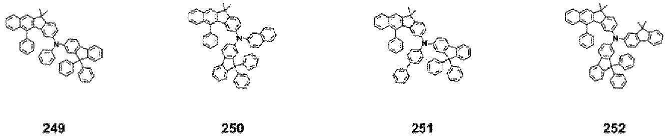

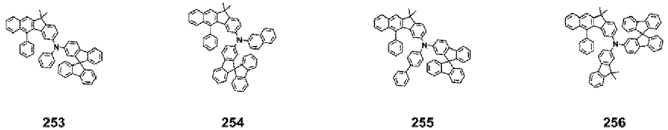

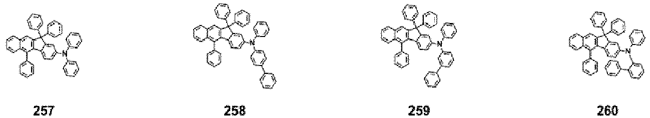

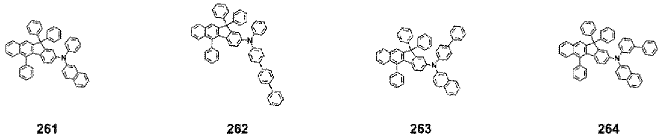

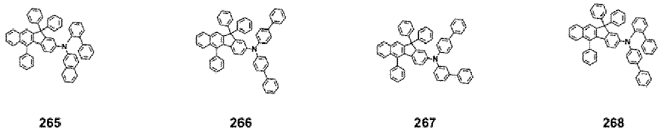

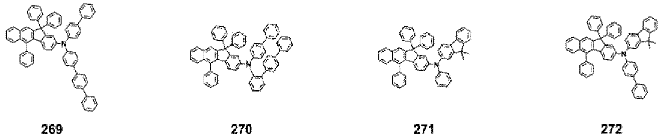

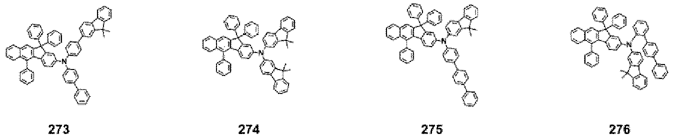

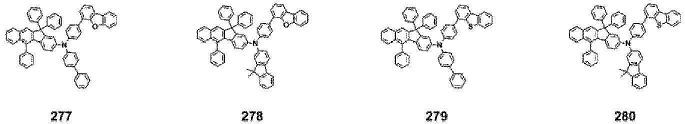

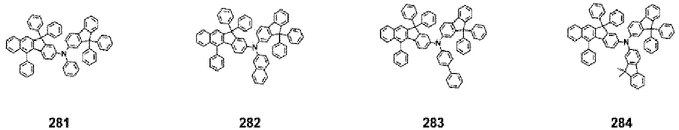

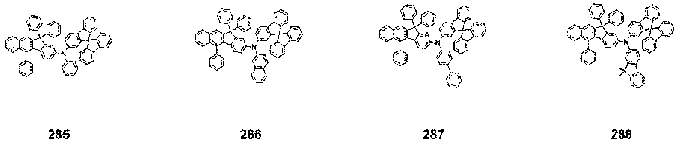

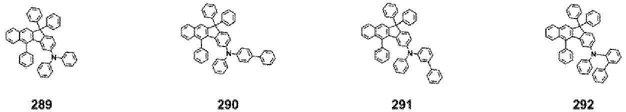

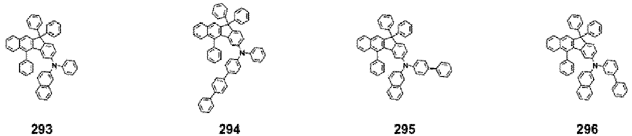

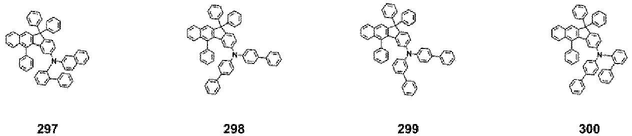

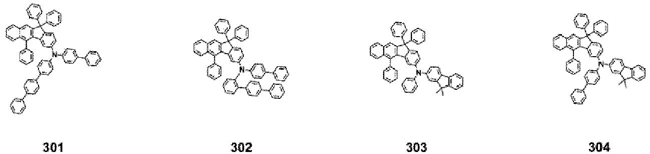

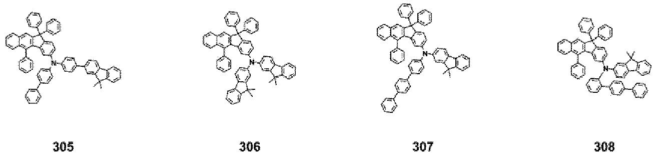

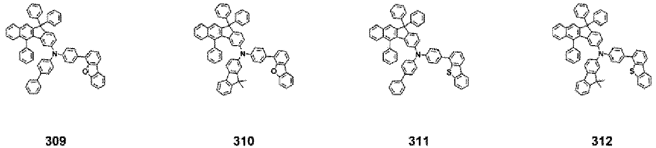

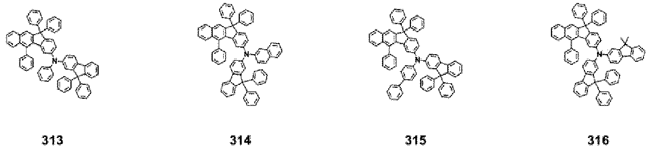

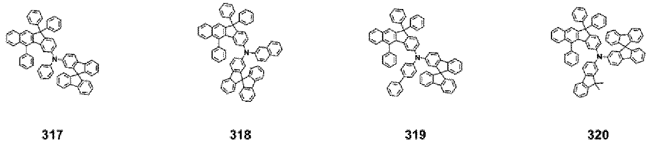

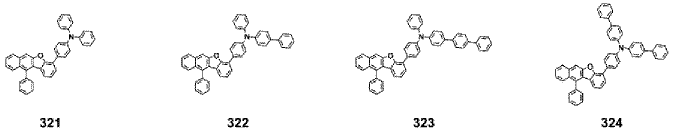

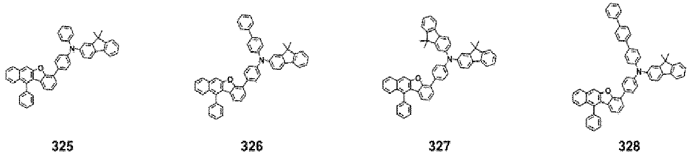

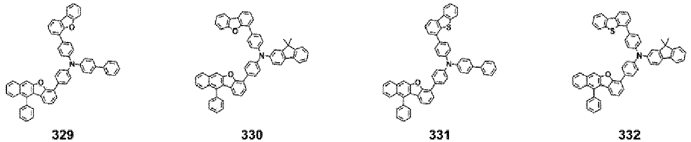

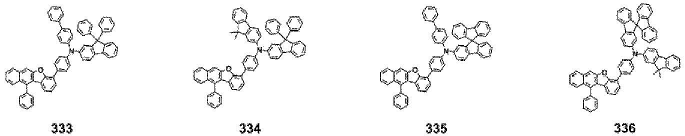

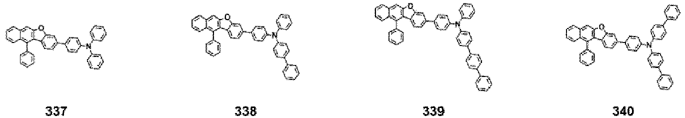

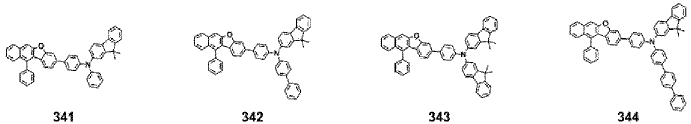

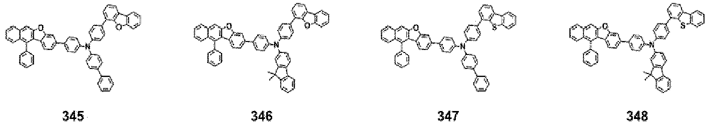

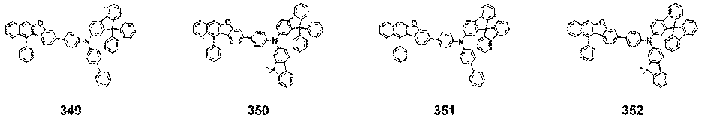

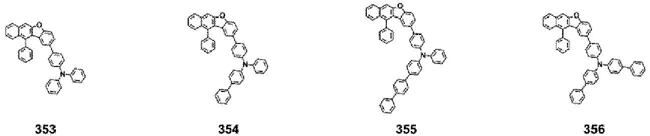

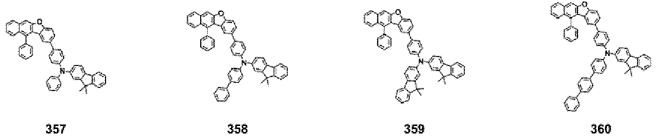

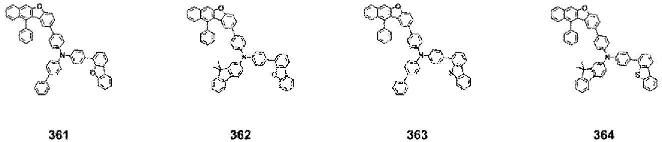

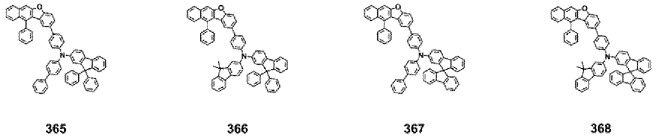

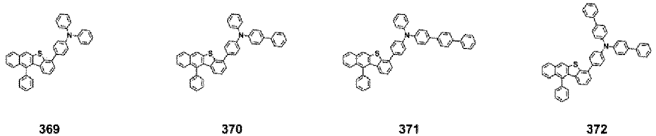

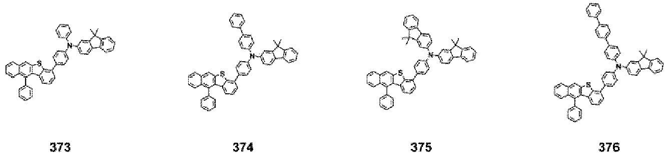

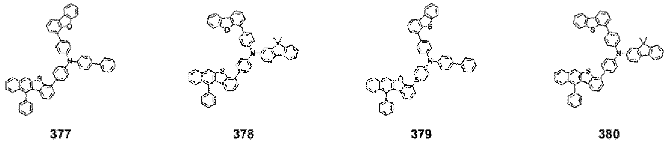

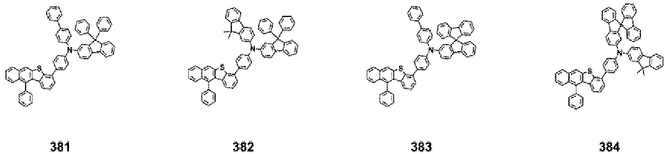

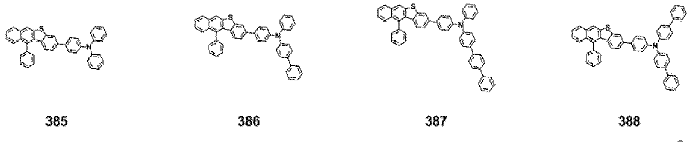

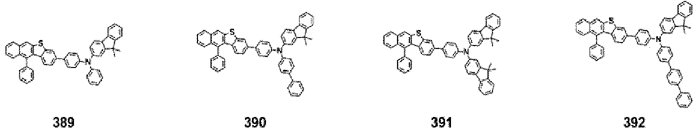

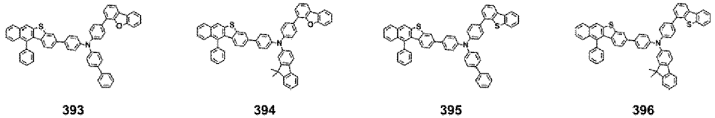

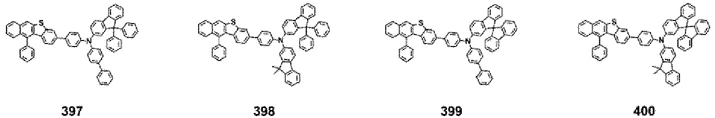

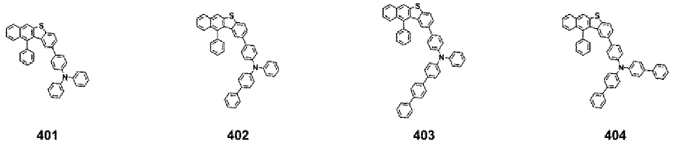

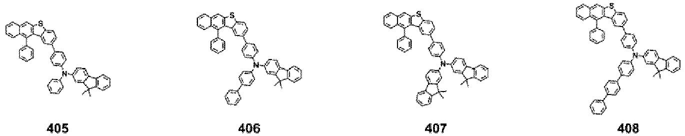

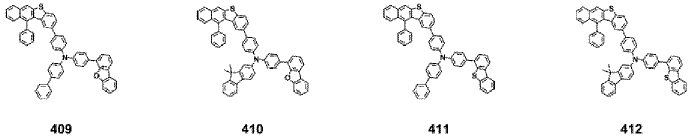

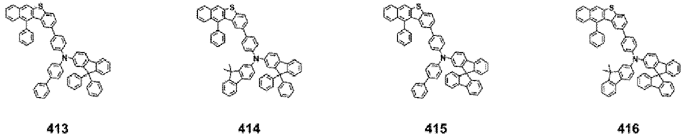

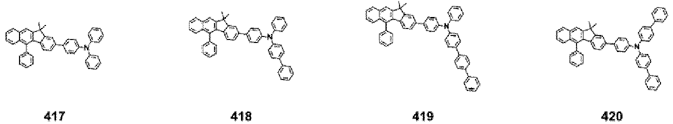

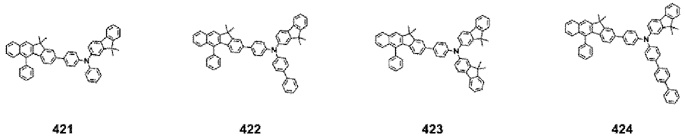

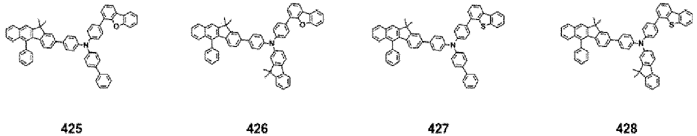

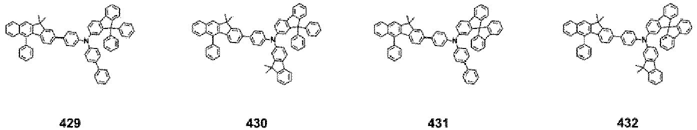

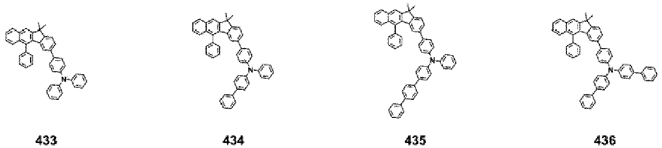

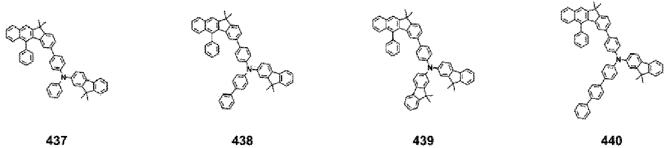

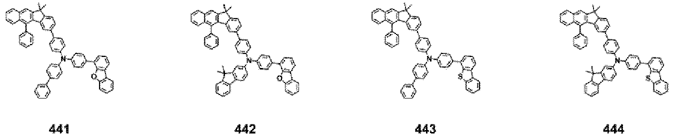

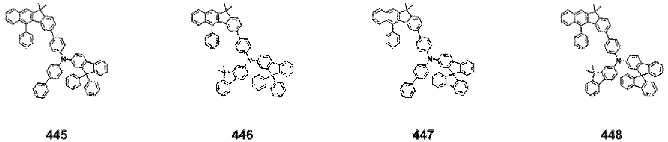

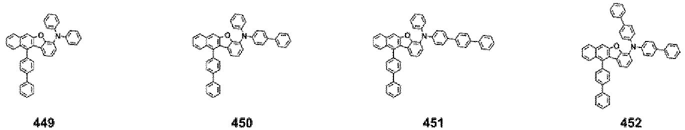

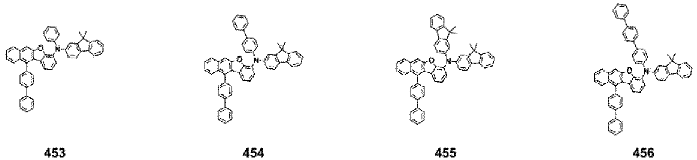

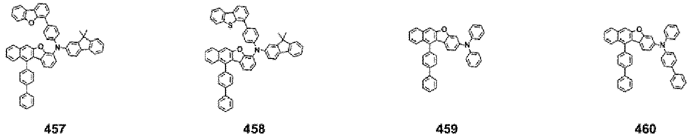

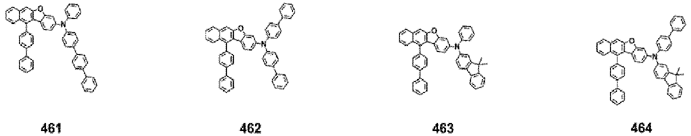

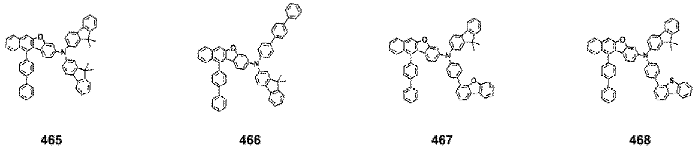

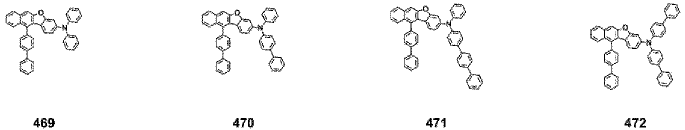

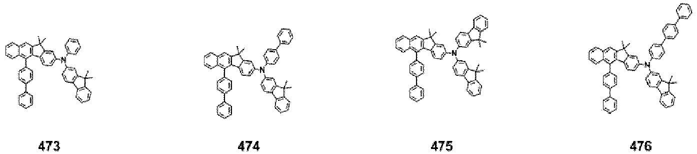

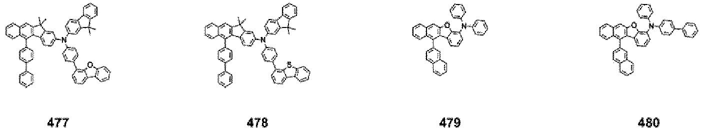

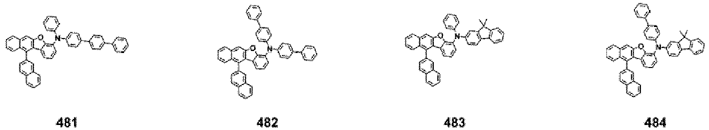

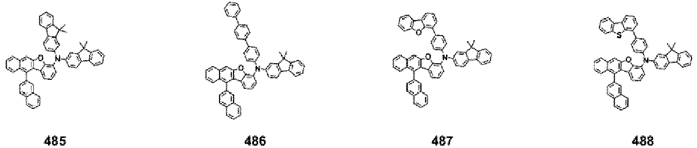

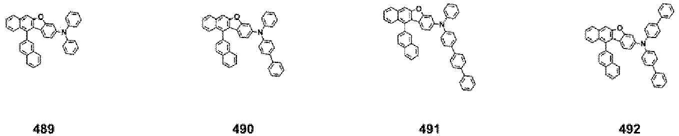

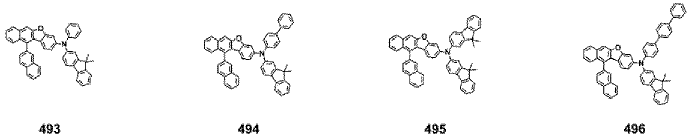

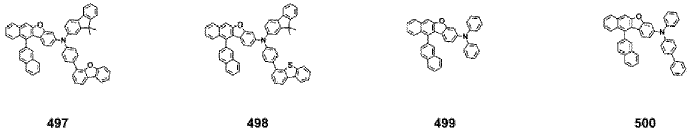

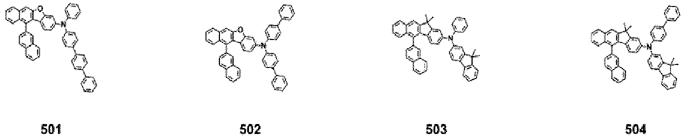

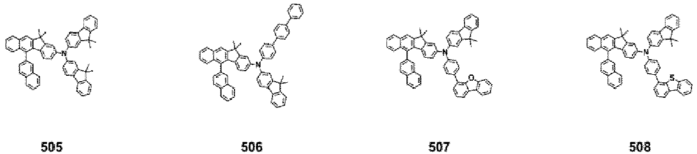

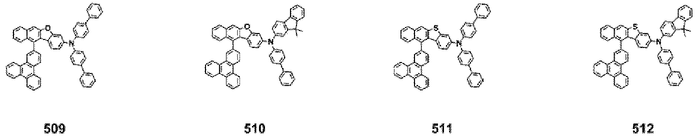

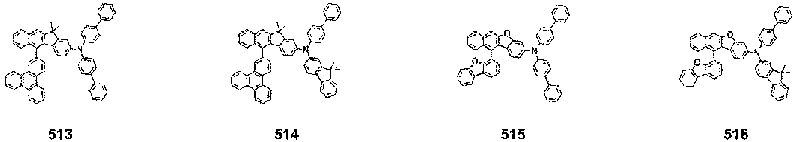

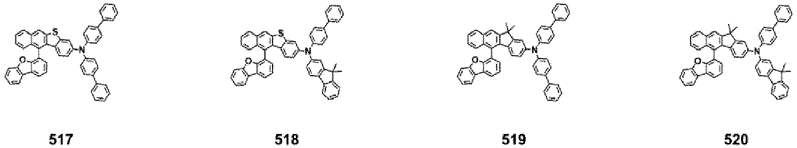

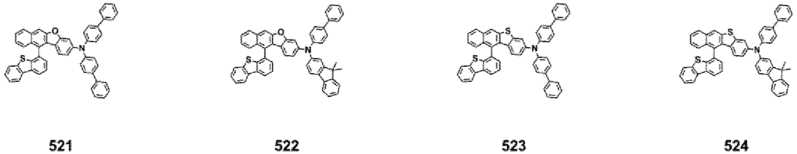

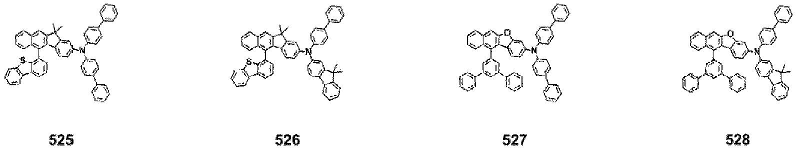

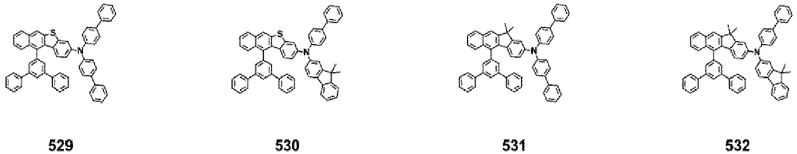

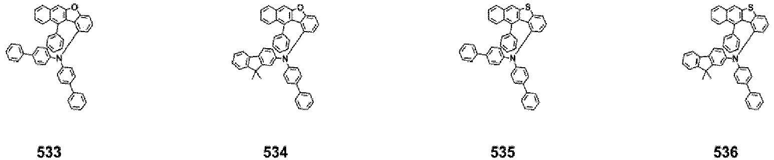

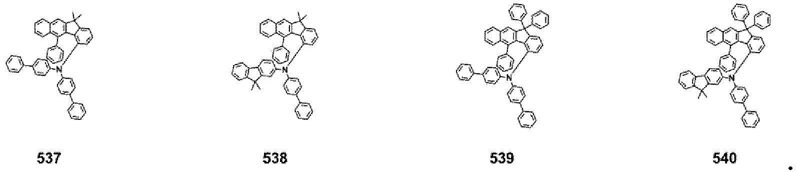

7. An organic light emitting device comprising:
a first electrode;
a second electrode; and
an organic material layer provided between the first electrode and the second electrode,
wherein the organic material layer includes the compound of any one of Claims 1 to 6.

8. The organic light emitting device of Claim 7, wherein the organic material layer includes a hole transfer layer, and the hole transfer layer includes the compound.

9. The organic light emitting device of Claim 7, wherein the organic material layer includes an electron blocking layer, and the electron blocking layer includes the compound.
